(19) Europäisches Patentamt
European Patent Office
Office européen des brevets

(11) EP 4 656 625 A1

(12) **EUROPEAN PATENT APPLICATION**
published in accordance with Art. 153(4) EPC

(43) Date of publication:
**03.12.2025 Bulletin 2025/49**

(21) Application number: **24747378.8**

(22) Date of filing: **26.01.2024**

(51) International Patent Classification (IPC):
***C07C 51/00*** *(2006.01)* ***B01J 31/22*** *(2006.01)*
***C07B 61/00*** *(2006.01)* ***C07C 53/02*** *(2006.01)*

(52) Cooperative Patent Classification (CPC):
**B01J 31/22; C07B 61/00; C07C 51/00; C07C 53/02**

(86) International application number:
**PCT/JP2024/002489**

(87) International publication number:
**WO 2024/158054 (02.08.2024 Gazette 2024/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **26.01.2023 JP 2023010364**

(71) Applicant: **National Institute of Advanced Industrial Science and Technology**
**Chiyoda-ku**
**Tokyo 100-8921 (JP)**

(72) Inventor: **KAWANAMI, Hajime**
**Tsukuba-shi, Ibaraki 305-8560 (JP)**

(74) Representative: **V.O.**
**P.O. Box 87930**
**2508 DH Den Haag (NL)**

(54) **FORMIC ACID PRODUCTION METHOD**

(57) A method for producing formic acid comprises reacting hydrogen and carbon dioxide in the presence of a catalyst, using either a reaction medium containing 10% by mass or less water, or no reaction medium. The catalyst comprises an organic iridium complex or an organic ruthenium complex. The reaction is conducted at a hydrogen partial pressure of at least 0.05 MPa and a carbon dioxide partial pressure of at least 0.05 MPa.

FIG. 1

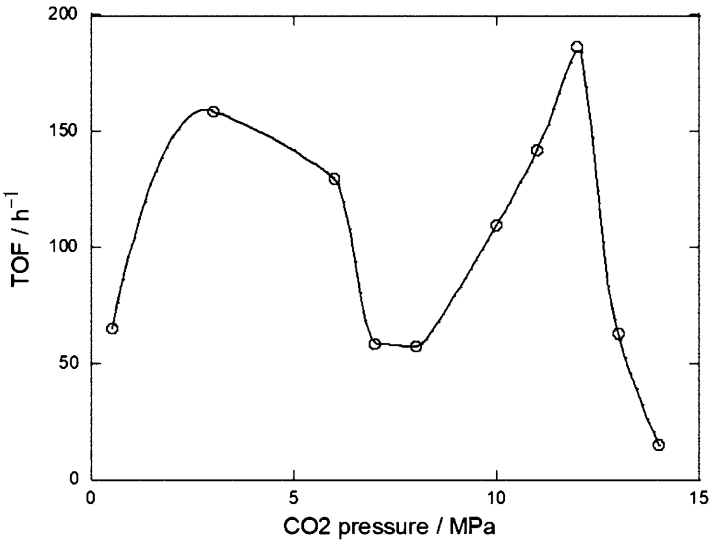


EP 4 656 625 A1

## Description

TECHNICAL FIELD

The present application relates to a method for producing formic acid from hydrogen and carbon dioxide using a catalyst.

Priority is claimed on Japanese Patent Application No. 2023-010364, filed on January 26, 2023, the content of which is incorporated herein by reference.

BACKGROUND ART

Hydrogen is recognized as a promising next-generation fuel, and various efforts have been made in Japan to establish a hydrogen-based society. Recent studies have demonstrated the dehydrogenation of formic acid using iridium catalysts to produce hydrogen and carbon dioxide (Patent Document 1). Additionally, high-pressure hydrogen and carbon dioxide-equivalent in volume to the formic acid-have been generated at pressures up to 157 MPa without compressors (Patent Document 2, Non-Patent Document 1). While overseas applications include fuel cell power generation using this high-pressure gas mixture, challenges remain in recovering $CO_2$, limiting the use of formic acid as a hydrogen carrier in Japan.

The inventors of the present application have developed a method of easily separating hydrogen and carbon dioxide by using a high-pressure condition of gas obtained from formic acid (Patent Document 1, Patent Document 2, and Non Patent Document 2). While the hydrogen is used as energy, the development of a method for effectively utilizing the carbon dioxide is expected. One of the methods for effectively utilizing the carbon dioxide is a technology for regenerating the formic acid from hydrogen and carbon dioxide. When the formic acid is regenerated from hydrogen obtained from renewable energy such as solar energy carbon dioxide separately recovered from a high-pressure gas obtained from formic acid, the hydrogen can be stored in the form of formic acid without discharging the carbon dioxide.

However, the reaction of obtaining the formic acid from the hydrogen and the carbon dioxide has a high energy barrier. Therefore, by generating a formate from hydrogen and carbon dioxide under alkaline conditions, the energy barrier is lowered (Non Patent Document 3). In order to obtain the formic acid from the formate obtained from hydrogen and carbon dioxide, the formate is neutralized with sulfuric acid or the like, and then distilled (Non Patent Document 4). In order to eliminate such a complicated step, there is a demand for a method of producing formic acid which is stable, instead of the formate from hydrogen and carbon dioxide.

Citation List

Patent Documents

Patent Document 1: Japanese Patent No. 6502091
Patent Document 2: Japanese Patent No. 7370040

Non Patent Documents

Non Patent Document 1: Ligand Design for Catalytic Dehydrogenation of Formic Acid to Produce High-pressure Gas under Base-Free Conditions, H. Kawanami, M. Iguchi, Y. Himeda, Inorganic Chemistry, Vol. 59, No. 7, Page 4191-4199, 2020.
Non Patent Document 2: National Research and Development Agency New Energy and Industrial Technology Development Organization, FY 2015 Results Report New Energy Venture Technology Innovation Project New Energy Venture Technology Innovation Project (Fuel Cell and Storage Battery) "Development of Energy Saving Hydrogen Boosting System Using Renewable Energy", November 14, 2017.
Non Patent Document 3: D. Wei, R. Sang, P. Sponholz, H. Junge, M. Beller, Nature Energy Vol. 7, 438-448, 2022.
Non Patent Document 4: Formic Acid Manufacture, W. L. Hardy, Industrial and Engineering Chemistry, Vol. 49, No. 7, Page 45A-46A, 1957.

SUMMARY OF INVENTION

Technical Problem

The present application has been made in view of such circumstances, and an object of the present invention is to provide a method for producing, from hydrogen and carbon dioxide, formic acid which is stable.

Solution to Problem

The inventors of the present application have found that an aqueous medium used in production of formic acid from hydrogen and carbon dioxide promotes a decomposition reaction of the formic acid into hydrogen and carbon dioxide. In addition, the inventors of the present application have also found that, when the aqueous medium is not used in the production of formic acid from hydrogen and carbon dioxide, the decomposition reaction of the formic acid into hydrogen and carbon dioxide is slowed down, and the generation of high-pressure gas is not accompanied. Furthermore, it has been also found that, when the hydrogen and the carbon dioxide are mixed at a high pressure under an anhydrous condition in the presence of various organic metal complex catalysts or various solid catalysts, the formic acid is produced. Therefore, it has been found that, when the hydrogen and the carbon dioxide are reacted at a high pressure of 0.5 MPa or more in the presence of various organic metal complex catalysts or various metal-supported solid catalysts under an anhydrous condition, that is, in a state of no aqueous medium or a medium having almost no water, the formic acid is efficiently produced. The present invention is achieved by utilizing and further developing these findings.

A formic acid production method according to an aspect of the present application is a method for producing formic acid from hydrogen and carbon dioxide in the presence of a catalyst, using a reaction medium in which a content of water is 10% by mass or less or without using a reaction medium, in which the catalyst contains an organic iridium complex and an organic ruthenium complex, and a partial pressure of the hydrogen is 0.05 MPa or more and a partial pressure of the carbon dioxide is 0.05 MPa or more.

A formic acid production method according to another aspect of the present application is a method for producing formic acid from hydrogen and carbon dioxide in the presence of a catalyst, using a reaction medium in which a content of water is 10% by mass or less or without using a reaction medium, in which the catalyst contains a support and one or more metals of chromium, manganese, iron, cobalt, nickel, copper, zinc, molybdenum, ruthenium, rhodium, palladium, silver, tungsten, rhenium, osmium, iridium, platinum, and gold, the metals being supported on the support, and a partial pressure of the hydrogen is 0.05 MPa or more and a partial pressure of the carbon dioxide is 0.05 MPa or more.

Advantageous Effects of Invention

In the present application, hydrogen and carbon dioxide at a predetermined pressure or higher are reacted in the presence of a predetermined catalyst, using a reaction medium in which a content of water is 10% by mass or less or without using a reaction medium. Therefore, according to the present application, formic acid, which is not a formate and is stable, can be produced from the hydrogen and the carbon dioxide.

BRIEF DESCRIPTION OF DRAWINGS

[FIG. 1] A graph representing a relationship between a partial pressure of carbon dioxide and TOF in Example 4.
[FIG. 2] A graph representing a relationship between a reaction time and TON in Example 5.
[FIG. 3] A graph representing a relationship between a partial pressure of hydrogen and a partial pressure of carbon dioxide, and TON in Example 7.
[FIG. 4] A graph representing a relationship between a reaction time and TON in Example 7.

DESCRIPTION OF EMBODIMENTS

With the formic acid production method according to the embodiment of the present application, formic acid is produced from hydrogen and carbon dioxide in the presence of a predetermined catalyst, using a reaction medium in which a content of water is 10% by mass or less or without using a reaction medium. The produced formic acid is not a formate and is stable. The term "not a formate" means $HCO_2H$, and means that it is not a salt such as $HCO_2^-Na^+$. The stable formic acid refers to a formic acid in which a rate of decrease in concentration of the formic acid is 1% by mass or less even after 72 hours at 25°C under atmospheric pressure in a condition without a catalyst or the like in a glass container or a resin container. The predetermined catalyst may contain an organic iridium complex or an organic ruthenium complex. In addition, the predetermined catalyst may contain a support and a predetermined metal supported on the support. The predetermined metal is one or more kinds of chromium, manganese, iron, cobalt, nickel, copper, zinc, molybdenum,

ruthenium, rhodium, palladium, silver, tungsten, rhenium, osmium, iridium, platinum, and gold. The predetermined metal may be an alloy.

It is preferable that these catalysts are uniformly dissolved or dispersed in a reaction medium which contains almost no water, that is, a reaction medium in which the content of water is 10% by mass or less. Here, the content of water means a proportion of a contained mass of water with respect to the total mass of the reaction medium. In addition, the "reaction medium which contains almost no water" includes a reaction medium having a content of water of 0% by mass, that is, a reaction medium which does not contain water, as well as the "reaction medium in which a content of water is 10% by mass or less". In order to suppress the produced formic acid from being re-decomposed in the presence of the catalyst to generate hydrogen and carbon dioxide, the content (content ratio) of water with respect to the total mass of the reaction medium is preferably 0% by mass or more and 5% by mass or less, more preferably 0% by mass or more and 1% by mass or less, and still more preferably 0% by mass or more and 0.5% by mass or less. In addition, an organic compound liquid, a subcritical fluid, a supercritical fluid, or the like can be used as the reaction medium.

In addition, these catalysts may be dispersed in or come into contact with the reaction medium which contains almost no water, as a solid or a gel in which the organic iridium complex and the organic ruthenium complex are supported by a polymer compound, or as a solid in various forms such as a powder, a wire shape, and a film shape. Furthermore, these catalysts may be used in combination. When producing the formic acid without using the reaction medium, it is sufficient that the hydrogen and the carbon dioxide are brought into contact with these catalysts. In addition, the origin and purity of the hydrogen and the carbon dioxide to be used are not particularly limited. When producing the formic acid without using the reaction medium, the carbon dioxide may be a subcritical fluid or a supercritical fluid depending on the partial pressure of the carbon dioxide, and this fluid of carbon dioxide functions as a reaction medium.

Examples of the organic iridium complex and the organic ruthenium complex include those represented by General Formula (1). Here, M is iridium or ruthenium. A and B are each independently a ligand containing nitrogen, carbon, oxygen, or sulfur as a coordinating atom to M. A and B may be the same or different from each other. A⌒B is a bidentate ligand coordinated to M. L is an aromatic anion ligand or an aromatic ligand. An aromatic ring of the aromatic anionic ligand or the aromatic ligand may have one or more substituents. Z is an optional ligand or is not present. $[C]^{n-}$ is a counterion when $n \neq 0$, or is not present when $n = 0$. m and n are integers.

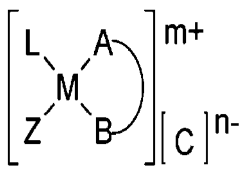

(1)

Examples of $[C]^{n-}$ which is an anion include a hexafluorophosphate ion ($PF_6^-$), a difluorophosphate ion ($F_2PO_2^-$), a tetrafluoroborate ion ($BF_4^-$), a bis(fluorosulfonyl)imide ion, a bis(trifluoromethanesulfonyl)imide ion, a hydroxide ion ($OH^-$), an acetate ion, a carbonate ion, a phosphate ion, a sulfate ion, a nitrate ion, halide ions such as a fluoride ion, a chloride ion, a bromide ion, and an iodide ion, hypohalate ions such as a hypofluorite ion, a hypochlorite ion, a hypobromite ion, and a hypoiodite ion, halate ions such as a fluorite ion, a chlorite ion, a bromite ion, and an iodite ion, perhalate ions such as a perfluorate ion, a perchlorate ion, a perbromate ion, and a periodate ion, a trifluoromethanesulfonate ion ($OSO_2CF_3^-$), a tetrakis(pentafluorophenyl)borate ion $[B(C_6F_5)_4^-]$, and a combination of two or more of these ions.

Examples of $[C]^{n-}$ which is a cation include various metal ions such as a lithium ion, a magnesium ion, a sodium ion, a potassium ion, a calcium ion, a barium ion, a strontium ion, a yttrium ion, a scandium ion, and a lanthanoid ion, a hydrogen ion, and a combination of two or more of these ions.

Examples of the substituent substituting the aromatic ring of L which is an aromatic anion ligand or an aromatic ligand include a hydroxy group (-OH), a nitro group ($-NO_2$), a halogen group (-X), a carboxyl group (-COOH), a sulfo group ($-SO_3H$), and an alkyl group, an alkoxy group (-OR), an alkylthio group (-SR), an amino group (-NRR'), an alkylamino group, an amide group (-CONRR'), an ester group (-COOR), or a phenyl group, which may have one or more substituents. When a plurality of substituents substituting the aromatic ring of L are present, these substituents may be the same or different from each other.

The organic iridium complex or the organic ruthenium complex represented by General Formula (1) is preferably represented by any one of General Formulae (2) to (6) and General Formulae (2') to (6'). In the organic iridium complex or the organic ruthenium complex represented by any one of General Formulae (2) to (6) and General Formulae (2') to (6'), $[C]^{n-}$ represents a counterion, but C other than $[C]^{n-}$, that is, C coordinated to M represents carbon. In addition, N and O coordinated to M each represent nitrogen and oxygen.

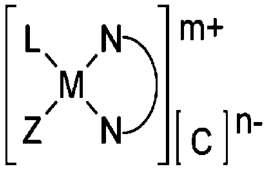

(2)

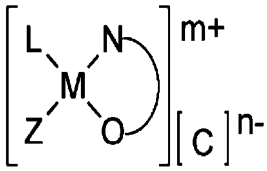

(3)

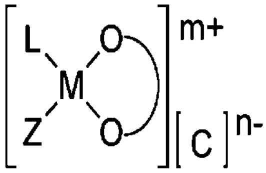

(4)

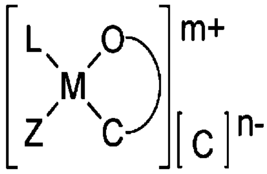

(5)

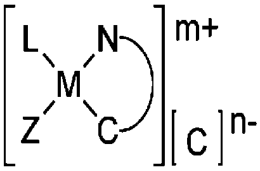

(6)

The organic iridium complex and the organic ruthenium complex represented by General Formula (1) are more preferably represented by any one of General Formulae (7) and (8) and General Formulae (7') and (8'). That is, L is more preferably a pentamethylcyclopentadienyl ligand or a p-cymene ligand. Q is nitrogen (N), oxygen (O), or carbon (C).

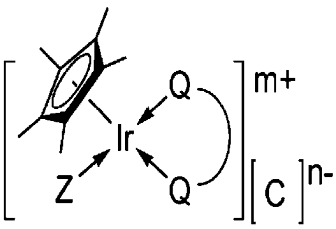

(7)

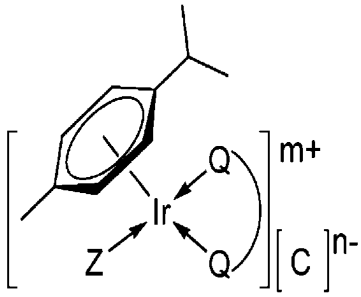

(8)

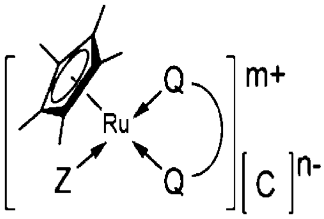

(7')

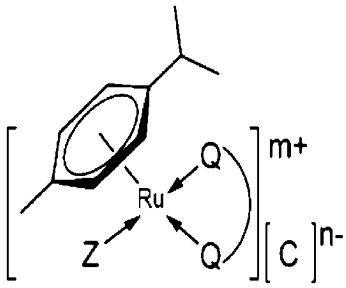

(8')

In the organic iridium complex and the organic ruthenium complex represented by any one of General Formulae (1) to (8) and General Formulae (7') and (8'), examples of the ligand Z include a water molecule, a hydride ion, an alkoxide ion, a hydroxide ion, a halide ion, a carbonate ion, a trifluoromethanesulfonate ion, a sulfate ion, a nitrate ion, a formate ion, and an acetate ion. Examples of the alkoxide ion include alkoxide ions derived from methanol, ethanol, n-propyl alcohol,

isopropyl alcohol, n-butyl alcohol, sec-butyl alcohol, isobutyl alcohol, tert-butyl alcohol, and the like.

The organic iridium complex represented by General Formula (7) and the organic ruthenium complex represented by General Formula (7') are preferably represented by any one of General Formulae (9) to (12) and General Formulae (9') to (12'). Here, $X_7$ to $X_{21}$ and $X^{22}$ to $X^{25}$ are each independently nitrogen, carbon, oxygen, or sulfur. When $X_h$ (h is an integer of 7 or more and 21 or less) or $X^i$ (i is an integer of 22 or more and 25 or less) is oxygen or sulfur, $R_j$ (j is an integer of 1 or more and 21 or less) and $R^k$ (k is an integer of 22 or more and 25 or less) bonded to $X_h$ or $X^i$ are not present. $Y_1$ to $Y_6$, $Y^7$, and $Y^8$ are each independently nitrogen or carbon.

$R_1$ to $R_{21}$ and $R^{22}$ to $R^{26}$ are each independently a hydrogen atom, a hydroxy group, a nitro group, a halogen group, a carboxyl group, a sulfo group, or an alkyl group, an alkoxy group, an alkylthio group, an amino group, an alkylamino group, an amide group, an ester group, or a phenyl group, which may have one or more substituents. The substituent is preferably a hydroxy group or an oxyanion group ($-O^-$). Adjacent $R_1$ to $R_{21}$ and $R^{22}$ to $R^{26}$ may form a ring. A bond between elements forming the ligand is a single bond or a double bond. The heterocyclic ring may be an aromatic ring or a non-aromatic ring. Q in General Formulae (12) and (12') is oxygen, sulfur, or selenium.

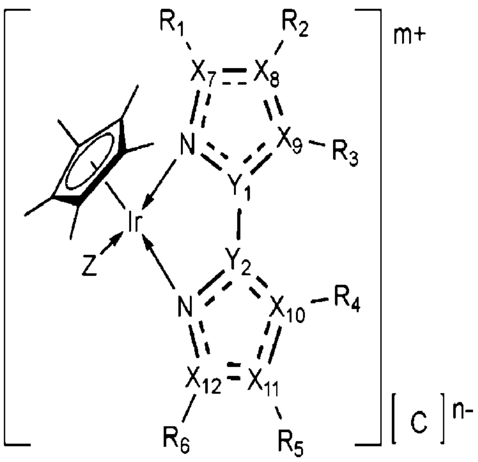

(9)

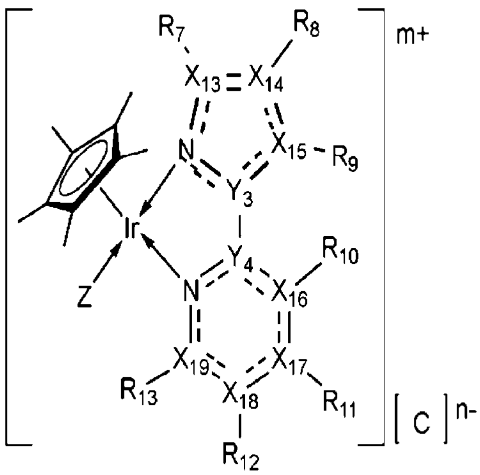

(10)

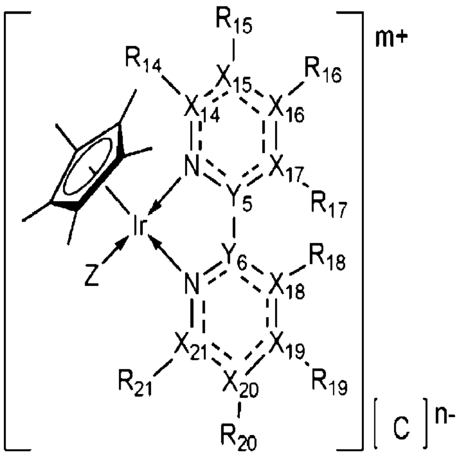

(11)

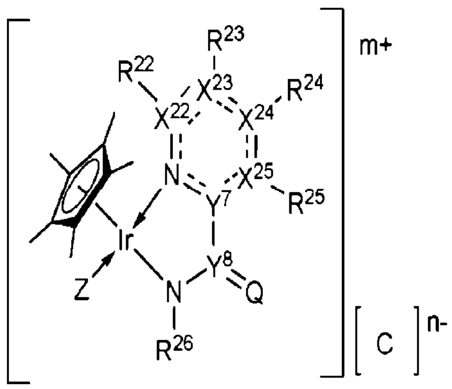

(12)

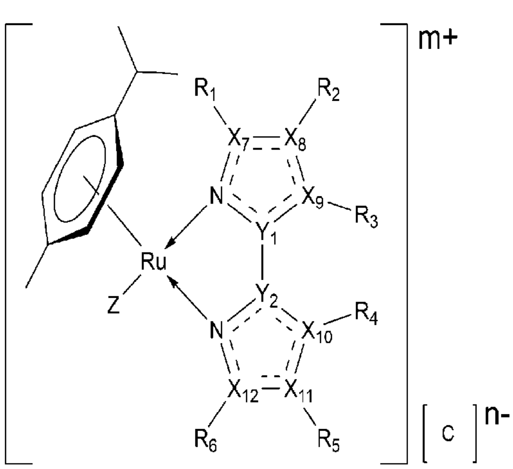

( 9' )

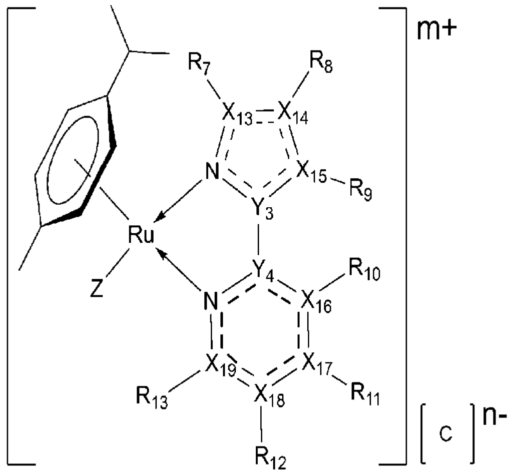

( 10' )

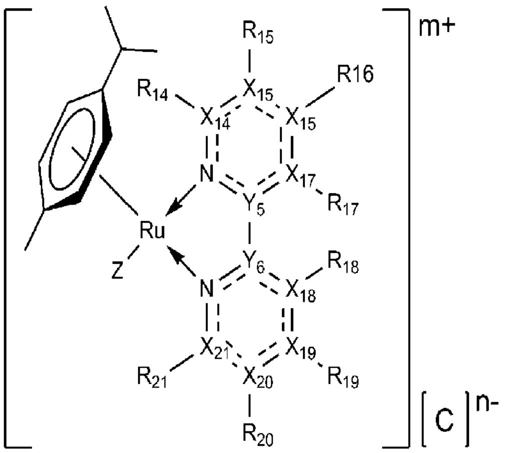

( 11' )

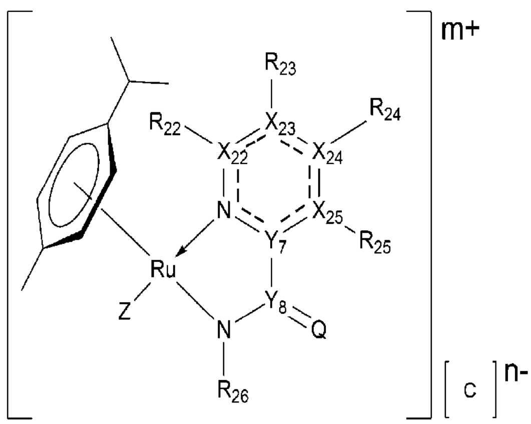

( 12' )

In Formulae (9) to (12) and (9') to (12'), the ring containing N is preferably an aromatic heterocyclic ring. $R_1$ to $R_{21}$ and $R^{22}$ to $R^{25}$ are each independently preferably an electron-donating group; and more preferably a hydrogen atom, a hydroxy group, an amino group, a monoalkylamino group having 1 to 6 carbon atoms, a dialkylamino group having 2 to 12 carbon atoms, a nitrogen-containing saturated heterocyclic group having 3 to 6 carbon atoms (for example, an aziridinyl group, an azetidinyl group, a pyrrolidinyl group, a piperidyl group, or the like), or an alkoxy group having 1 to 3 carbon atoms. $R^{26}$ is preferably an electron-donating group, more preferably an aromatic hydrocarbon group having 6 to 10 carbon atoms, and still more preferably a phenyl group or a naphthyl group.

As the counterion $[C]^{n-}$, a sulfate anion, a chloride anion, a hexafluorophosphate anion, a nitrate anion, a tetrafluoroborate anion, or a bis(trifluoromethanesulfonate)imide anion is preferable.

In Formulae (9) and (9'), $X_8$ is preferably a nitrogen atom and $X_{11}$ is also preferably a nitrogen atom.

In Formulae (10) and (10'), $X_{14}$ is preferably a nitrogen atom and $X_{16}$ to $X_{19}$ are preferably carbon atoms.

In Formulae (11) and (11'), $X_{14}$ to $X_{16}$ are preferably carbon atoms, $X_{17}$ is preferably a carbon atom or a nitrogen atom, $X_{18}$ is preferably a carbon atom or a nitrogen atom, and $X_{19}$ to $X_{21}$ are preferably carbon atoms.

In Formulae (12) and (12'), $X^{22}$ to $X^{25}$ are preferably carbon atoms.

The organic iridium complex and the organic ruthenium complex may be supported by a polymer compound with a covalent bond, an ionic bond, or the like (hereinafter, also referred to as "immobilized catalyst"). Examples of the polymer compound include one or more of polystyrene, polyethylene, polyethyleneimine, polyacrylic acid, poly(methyl methacrylate), polyvinyl alcohol, polyvinylpyrrolidone, polyethylene glycol, polyester, polyamide, polylactic acid, and polylysine. Among these, the polymer compound is preferably one or more of polyethyleneimine, polyacrylic acid, poly(methyl acrylate), polyvinyl alcohol, polyvinylpyrrolidone, or polylysine. In the immobilized catalyst, the organic iridium complex or the organic ruthenium complex is preferably bonded to a side chain of the polymer compound. In addition, in the immobilized catalyst, the organic iridium complex and the organic ruthenium complex may form a crosslinking structure which crosslinks these polymer compounds. When the polymer compound is polyethyleneimine, the organic iridium

complex and the organic ruthenium complex are preferably represented by Formula (11); and it is more preferable that at least one of $R_{14}$ to $R_{17}$ in Formula (11) is bonded to a secondary amino group of a polyethyleneimine molecule through a monoaminoalkylene group having 1 to 10 carbon atoms, and at least one of $R_{18}$ to $R_{21}$ in Formula (11) is bonded to a secondary amino group of another polyethyleneimine molecule through a monoaminoalkylene group having 1 to 10 carbon atoms. When the polymer compound is polyacrylic acid, the organic iridium complex and the organic ruthenium complex are preferably represented by Formulae (11) and (11'); and it is more preferable that at least one of $R_{14}$ to $R_{17}$ in Formulae (11) and (11') is bonded to a carboxy group of a polyacrylic acid molecule through a diaminoalkylene group having 1 to 10 carbon atoms, and at least one of $R_{18}$ to $R_{21}$ in Formulae (11) and (11') is bonded to a carboxy group of another polyacrylic acid molecule through a diaminoalkylene group having 1 to 10 carbon atoms. In these polymers, a crosslinking structure other than the crosslinking structure derived from the organic iridium complex or the organic ruthenium complex may be provided. Examples of a crosslinking agent forming such a crosslinking structure include methylenebisacrylamide. A mass of iridium (atomic weight: 192.2) is preferably 0.001% to 30% by mass, more preferably 0.01% to 20% by mass, and most preferably 0.05% to 10% by mass with respect to the total mass of the immobilized catalyst. When the mass proportion of iridium as a mass proportion of a constitutional unit having an organic iridium complex is within the above-described range, it is possible to provide a reaction field for producing formic acid. A mass of ruthenium (atomic weight: 101.1) is preferably 0.001% to 30% by mass, more preferably 0.01% to 20% by mass, and most preferably 0.05% to 10% by mass with respect to the total mass of the immobilized catalyst. When the mass proportion of ruthenium as a mass proportion of a constitutional unit having an organic ruthenium complex is within the above-described range, it is possible to provide a reaction field for producing formic acid.

The above-described iridium-containing moiety constituting the organic iridium complex is preferably represented by any one of General Formulae (13) to (19).

The above-described ruthenium-containing moiety constituting the organic ruthenium complex is preferably represented by any one of General Formulae (13) to (19).

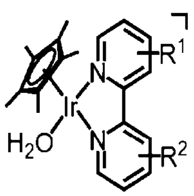
(13)

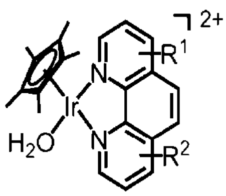
(14)

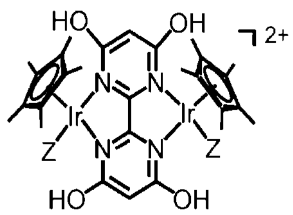
(15)

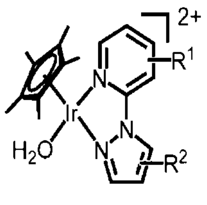
(16)

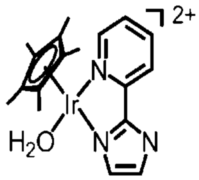
(17)

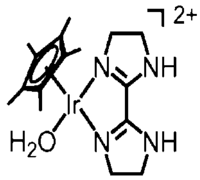
(18)

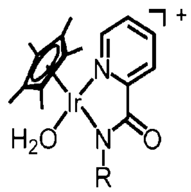
(19)

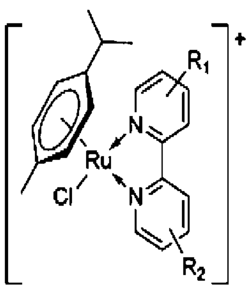
( 13')

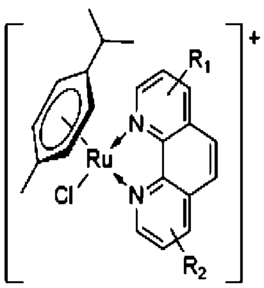
( 14')

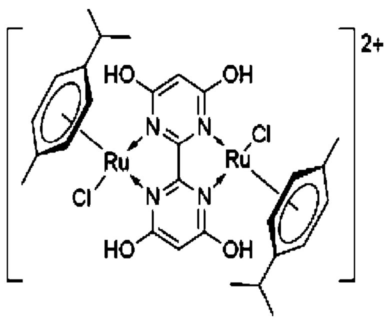
( 15')

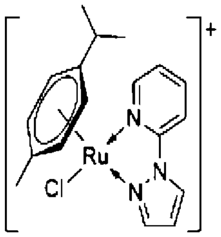 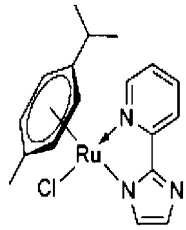 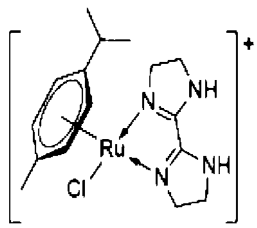 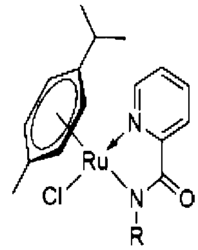

( 16' ) ( 17' ) ( 18' ) ( 19' )

R, $R^1$, and $R^2$ are each independently a hydrogen atom, a hydroxy group, a nitro group, a halogen group, a carboxyl group, a sulfo group, or an alkyl group, an alkoxy group, an alkylthio group, an amino group, an alkylamino group, an amide group, an ester group, or a phenyl group, which may have one or more substituents. Adjacent R, $R^1$, and $R^2$ may form a ring.

The catalyst may contain a support and a predetermined metal supported on the support (hereinafter, also referred to as "solid catalyst"). The predetermined metal is one or more kinds selected from the group consisting of chromium, manganese, iron, cobalt, nickel, copper, zinc, molybdenum, ruthenium, rhodium, palladium, silver, tungsten, rhenium, osmium, iridium, platinum, and gold. The predetermined metal may be an alloy.

Examples of the combination of the predetermined metal and the support include Pd/C, Pd-Au/C, Pd-Ag/C; Pd/rGO, Pd-Au/rGO, Pd-Ag/rGO; and Pd/PDA/rGO, Pd-Au/PDA/rGO, and Pd-Ag/PDA/rGO. Here, Pd/C is a support consisting of carbon, which supports palladium, Pd-Au/C is a support consisting of carbon, which supports an alloy of palladium and gold, and Pd-Ag/C is a support consisting of carbon, which supports an alloy of palladium and silver. Pd/rGO is a support consisting of graphene oxide, which supports palladium. Pd-Au/rGO is a support consisting of graphene oxide, which supports an alloy of palladium and gold. Pd-Ag/rGO is a support consisting of graphene oxide, which supports an alloy of palladium and silver. Pd/PDA/rGO is a support consisting of graphene oxide and para-aminobenzene, which supports palladium. Pd-Au/PDA/rGO is a support consisting of graphene oxide and para-aminobenzene, which supports an alloy of palladium and gold. Pd-Ag/PDA/rGO is a support consisting of graphene oxide and para-aminobenzene, which supports an alloy of palladium and silver. In the alloy of palladium and gold, a mass proportion represented by palladium:gold is preferably 1:4 to 4:1, more preferably 2:3 to 3:2, and still more preferably 1:1. In the alloy of palladium and silver, a mass proportion represented by palladium: silver is preferably 2:3 to 3:2 and more preferably 1:1. A mass proportion represented by [metal or alloy]:[total of supports] is preferably 1:99 to 10:90, more preferably 3:97 to 7:93, and still more preferably 5:95.

When the formic acid is produced using a support and a catalyst containing a predetermined metal supported on a support, the predetermined metal is preferably one or more kinds of gold, silver, or platinum. Examples of the support include one or more kinds of carbon materials such as carbon and a carbon resin, alumina, silica, zeolite, mesoporous silica, titania, zirconia, magnesia, and ceria. Among these, the support is preferably one or more kinds of carbon materials, alumina, and silica. A particle diameter of the metal to be supported is preferably 1 nm or more and 1 $\mu$m or less, and more preferably 5 nm or more and 500 $\mu$m or less.

Examples of a reaction medium when producing the formic acid by reacting the hydrogen with the carbon dioxide include pentane, hexane, heptane, octane, nonane, decane, toluene, styrene, xylene, mesitylene, methanol, ethanol, trifluoroethanol, pentafluoroethanol, propanol, isopropyl alcohol, hexafluoroisopropanol, butanol, isobutyl alcohol, pentanol, isopentyl alcohol, hexanol, cyclohexanol, 1,4-dioxane, tetrahydrofuran, methyl tetrahydrofuran, diethyl ether, ethylene glycol, ethylene glycol monoethyl ether, ethylene glycol monomethyl ether, acetic acid, trifluoroacetic acid, isobutyl acetate, isopropyl acetate, isopentyl acetate, ethyl acetate, butyl acetate, propyl acetate, acetone, methyl ethyl ketone, cyclohexanone, dimethyl carbonate, diethyl carbonate, ethylene carbonate, propylene carbonate, acetonitrile, N,N-dimethylformamide, N,N-dimethylacetamide, N,N-diethylformamide, N,N-diethylacetamide, tetramethylurea, tetraethylurea, dimethyl sulfoxide, dichloromethane, chloroform, dichloroethane, tetrachloroethane, chlorobenzene, dichlorobenzene, trichlorobenzene, phenol, dimethyl sulfoxide, and a combination of two or more of these solvents. Among these, in order to further exhibit the catalytic function of the organic iridium complex and the organic ruthenium complex, a reaction medium which dissolves the organic iridium complex and the organic ruthenium complex is preferable. A moisture content of these organic solvents is preferably 10% by mass.

A pressure of the hydrogen and a pressure of the carbon dioxide in the production of formic acid is not particularly limited; but in order to improve the efficiency, the pressure of the hydrogen, that is, a partial pressure of the hydrogen is 0.05 MPa or more, the pressure of the carbon dioxide, that is, a partial pressure of the carbon dioxide is 0.05 MPa or more, and the total pressure is 0.1 MPa or more. It is preferable that the partial pressure of the hydrogen is 0.5 MPa or more and the partial pressure of the carbon dioxide is 0.4 MPa or more. It is more preferable that the partial pressure of the hydrogen is 0.5 MPa or more, the partial pressure of the carbon dioxide is 1 MPa or more, and the total pressure is 1.5 MPa or more. It is

still more preferable that the partial pressure of the hydrogen is 0.5 MPa or more and the partial pressure of the carbon dioxide is 3 MPa or more. It is most preferable that the partial pressure of the hydrogen is 0.5 MPa or more, the partial pressure of the carbon dioxide is 7.2 MPa or more, and the total pressure is 8 MPa or more. The upper limit of the partial pressure of the hydrogen, the partial pressure of carbon dioxide, and the total pressure depends on the pressure resistance performance or the like of the reaction container, and for example, the total pressure can be adapted to 200 MPa or more. When the pressure is too high, the total pressure can be adapted to 160 MPa or more, more suitably 80 MPa or more, still more suitably 50 MPa or more, and most suitably 35 MPa or more. Specifically, the partial pressure of the hydrogen is preferably 0.05 MPa or more and 100 MPa or less, preferably 0.05 MPa or more and 80 MPa or less, preferably 0.05 MPa or more and 40 MPa or less, more preferably 0.05 MPa or more and 34.95 MPa or less, still more preferably 0.5 MPa or more and 34.5 MPa or less, even more preferably 0.5 MPa or more and 34.0 MPa or less, particularly preferably 0.5 MPa or more and 32.0 MPa or less, and most preferably 0.5 MPa or more and 27.8 MPa or less. The partial pressure of the carbon dioxide is preferably 0.05 MPa or more and 100 MPa or less, more preferably 0.4 MPa or more and 120 MPa or less, still more preferably 1 MPa or more and 160 MPa or less, particularly preferably 3 MPa or more and 165.05 MPa or less, and most preferably 7.2 MPa or more and 165.5 MPa or less. The total pressure is preferably 0.1 MPa or more and 200 MPa or less, more preferably 1.5 MPa or more and 200 MPa or less, still more preferably 6 MPa or more and 200 MPa or less, even more preferably 8 MPa or more and 200 MPa or less, particularly preferably 10 MPa or more and 200 MPa or less, and most preferably 12 MPa or more and 200 MPa or less.

Since the boiling point and melting point of formic acid change depending on the reaction pressure when the formic acid is produced by reacting hydrogen with carbon dioxide, a reaction temperature when the formic acid is produced is not particularly limited as long as the formic acid is produced. However, in order to suppress self-decomposition of the formic acid, when the reaction is supported out at a total pressure of 0.1 MPa or more, the reaction temperature is preferably 120°C or lower; but since the boiling point of the formic acid at 0.1 MPa is 101°C or lower, the reaction temperature is preferably 101°C or lower. When the reaction is further supported out under other pressure conditions, the reaction temperature is preferably equal to or lower than the boiling point of the formic acid. In addition, in order to improve the efficiency of producing the formic acid, when the reaction is supported out at a total pressure of 0.1 MPa, the reaction temperature is preferably 8.4°C or higher, which is the melting point of the formic acid; and in a case where the reaction is further supported out under other conditions, the reaction temperature is preferably equal to or higher than the melting point of the formic acid. As a result, it is more preferable that the reaction temperature is 8.4°C or higher and 120°C or lower, 8.4°C or higher and 101°C or lower, 10°C or higher and 101°C or lower, or 32°C or higher and 101°C or lower.

The method for producing the formic acid may be a batch method, a semi-batch method, or a flow method.

A time (reaction time) for which the above-described total pressure is maintained within a predetermined range is not particularly limited, but the reaction can be suitably supported out without particularly specifying the time as long as the time is 10 minutes or more. It is good to secure a sufficient time for the production of formic acid to reach equilibrium. For example, the reaction time is preferably 10 minutes or more and 100 hours or less, more preferably 30 minutes or more and 100 hours or less, still more preferably 1 hour or more and 100 hours or less, and even more preferably 2 hours or more and 100 hours or less.

A temperature (reaction temperature) for which the above-described reaction time is maintained within a predetermined range is preferably 120°C or lower, more preferably 110°C or lower, still more preferably 30°C or higher and 100°C or lower, and particularly preferably 40°C or higher and 90°C or lower.

A TOF value is not particularly limited, but a catalytic rotation speed (TOF value) [$h^{-1}$] calculated by an expression described in Examples is preferably 0 $h^{-1}$ or more and less than 150 $h^{-1}$, more preferably 0 $h^{-1}$ or more and 140 $h^{-1}$ or less, still more preferably 0 $h^{-1}$ or more and 120 $h^{-1}$ or less, and particularly preferably 0 $h^{-1}$ or more and 100 $h^{-1}$ or less.

Example

Example 1: Organic iridium complex catalyst and organic ruthenium complex

10 mL of various reaction media shown in Tables 1 and 2 below were charged into a stainless steel autoclave having a capacity of 30 mL, which was provided with a pressure sensor and a temperature sensor, and the reaction media were degassed by bubbling with nitrogen. After degassing, 40 μL of a 5 mM solution of various organic iridium complexes was added to the autoclave. Next, the inside of the autoclave was pressurized with carbon dioxide up to 2 MPa, and stirred at room temperature for 90 minutes at 1,200 rpm, and then the carbon dioxide was depressurized to 0.5 MPa. The inside of the autoclave was pressurized with hydrogen at 0.5 MPa to set the total pressure to 1.0 MPa, and the hydrogen and the carbon dioxide were reacted by heating to 50°C.

Two hours after the start of the reaction, the autoclave was cooled with ice water to stop the reaction, and the inside of the autoclave was slowly returned to atmospheric pressure. The amount of formic acid produced in the solution remaining in the autoclave was measured by high performance liquid chromatography (HPLC) (the same applies hereinafter). By standardizing the amount of the catalyst charged (0.2 μmol) and the amount of formic acid produced

(mol/10 mL) by the reaction time, a catalytic rotation speed (TOF value) [$h^{-1}$] was calculated by the following expression (the same applies hereinafter). The results are listed in Tables 1 and 2.

TOF value [$h^{-1}$] = Amount of formic acid produced (mol/10 mL)/(0.2 μmol of amount of catalyst × 2 hours)

Chemical structures of the organic iridium complex used in Example 1 are shown below.

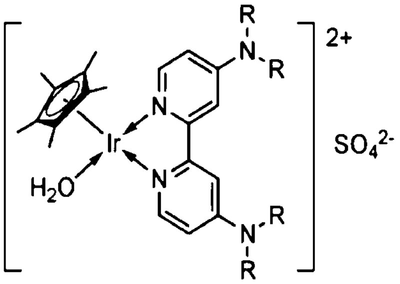

Compound 1a (R is hydrogen atom)

Compound 1b (R is methyl group)

Compound 1c (R is ethyl group)

Compound 1d (R is isopropyl group)

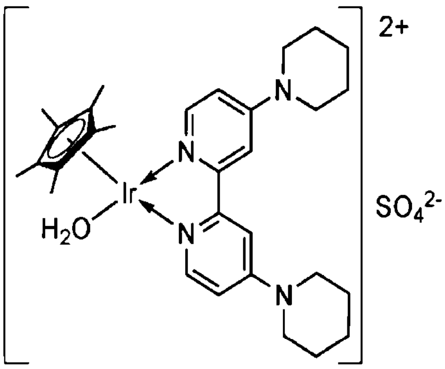

Compound 1e

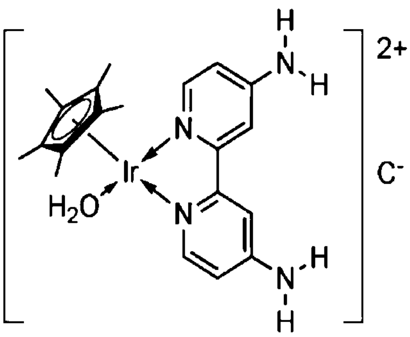

Compound 1f (C is hexafluorophosphate anion)

Compound 1g (C is nitrate anion)

Compound 1h (C is tetrafluoroborate anion)

Compound 1i (C is bis(trifluoromethanesulfonyl)imide anion)

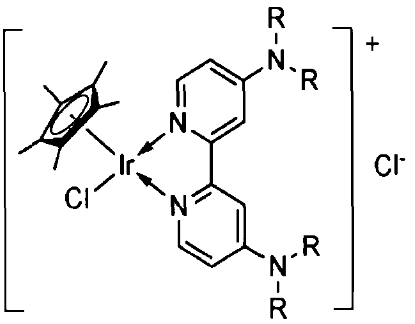

Compound 2a (R is hydrogen atom)

Compound 2b (R is methyl group)

Compound 2c (R is ethyl group)

Compound 2d (R is isopropyl group)

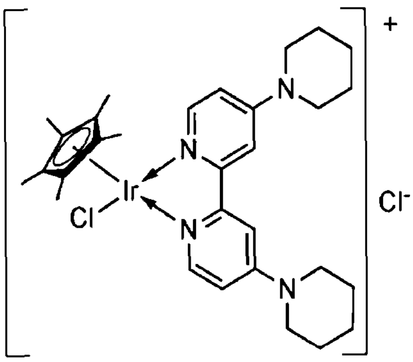

Compound 2e

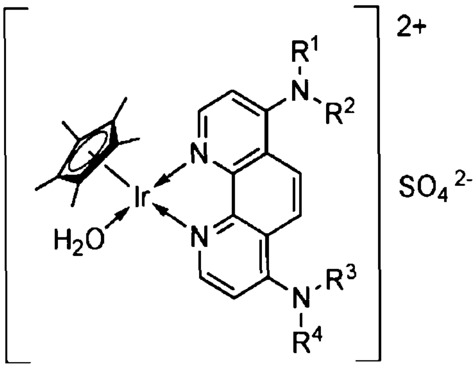

Compound 3a ($R^1$ to $R^4$ are hydrogen atom)

Compound 3b ($R^1$ to $R^4$ are methyl group)

Compound 3c ($R^1$ to $R^4$ are ethyl group)

Compound 3d ($R^1$ to $R^4$ are isopropyl group)

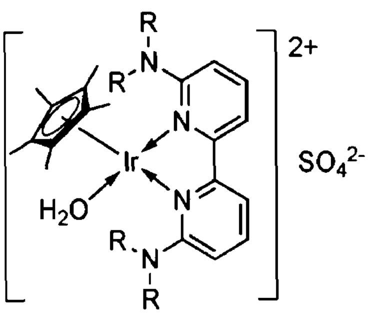

Compound 4a (R is hydrogen atom)

Compound 4b (R is methyl group)

Compound 4c (R is ethyl group)

Compound 4d (R is isopropyl group)

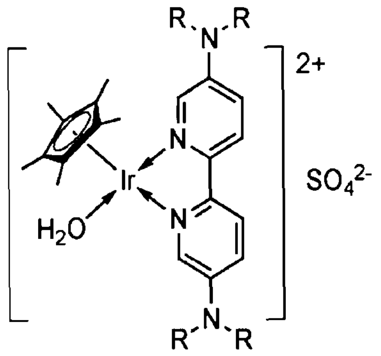

Compound 5a (R is hydrogen atom)

Compound 5b (R is methyl group)

Compound 5c (R is ethyl group)

Compound 5d (R is isopropyl group)

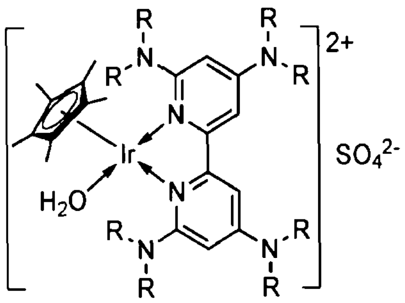

Compound 6a (R is hydrogen atom)

Compound 6b (R is methyl group)

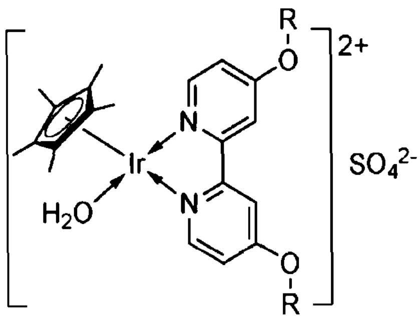

Compound 7a (R is hydrogen atom)

Compound 7b (R is methyl group)

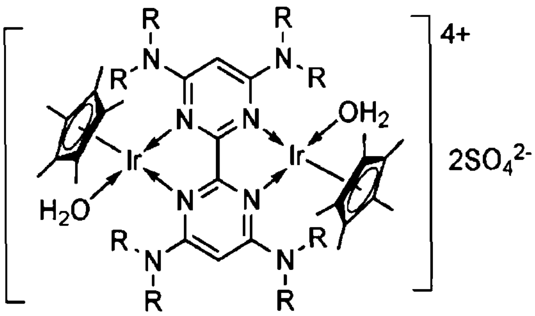

Compound 8a (R is hydrogen atom)

Compound 8b (R is methyl group)

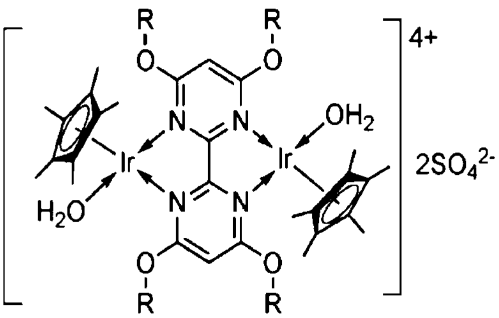

Compound 9a (R is hydrogen atom)

Compound 9b (R is methyl group)

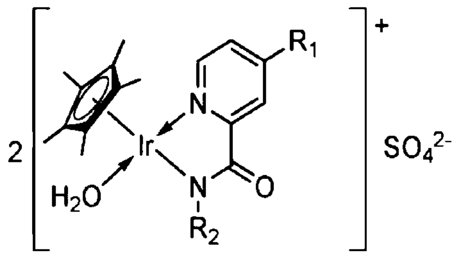

or

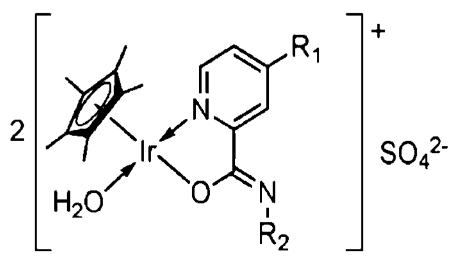

Compound 10a ($R_1$ is hydrogen atom and $R_2$ is phenyl group)

Compound 10b ($R_1$ is methyl group and $R_2$ is phenyl group)

Compound 10c ($R_1$ is hydroxy group and $R_2$ is phenyl group)

Compound 10d ($R_1$ is methoxy group and $R_2$ is phenyl group)

Compound 10e ($R_1$ is dimethylamino gruop and $R_2$ is phenyl group)

The compounds 1a to 1e, the compounds 2a to 2e, the compounds 3a to 3d, the compounds 4a to 4d, the compounds 5a to 5d, the compounds 6a and 6b, the compounds 7a and 7b, the compounds 8a and 8b, the compounds 9a and 9b, and the compounds 10a to 10e were synthesized based on the description in Inorganic Chemistry, 2020, 59, 7, 4191-4199. In addition, the compounds 1f to 1i were prepared by anion exchange by adding each of silver hexafluorophosphate (compound 1f), silver nitrate (compound 1g), silver tetrafluoroborate (compound 1h), or silver bis(trifluoromethanesulfonyl)imide (compound 1i) in the same amount as the compound 1a to a solution obtained by dissolving the compound 1a in a mixture of water and methanol.

[Table 1]

| Experimental No. | Catalyst | Reaction medium | TOF [$h^{-1}$] |
|---|---|---|---|
| 1 | Compound 1b | Water | 150 |
| 2 | | Methanol | 5 |
| 3 | | Ethanol | 1 |
| 4 | | 2-Propanol | 15 |
| 5 | | Ethylene glycol | 9 |
| 6 | | Hexafluoroisopropanol | 65 |
| 7 | | Trifluoroethanol | 1 |
| 8 | | Pentafluoroethanol | 5 |
| 9 | | Trifluoropropanol | 10 |
| 10 | | Propylene glycol monomethyl ether | 5 |
| 11 | | Dichloromethane | 2 |
| 12 | | Dichloroethane | 2 |
| 13 | | Tetrachloroethane | 5 |
| 14 | | Acetone | 1 |
| 15 | | Acetonitrile | 3 |
| 16 | | Dimethylformamide | 2 |
| 17 | | Dimethyl sulfoxide | 1 |
| 18 | | Dioxane | 2 |
| 19 | | Perfluoroisobutylene | 10 |
| 20 | | Octadecafluorooctane | 4 |
| 21 | | Eicosafluorononane | 2 |
| 22 | | Hexafluoromethyl ether | 8 |
| 23 | | Perfluorotriethylamine | 6 |
| 24 | | Perfluorotoluene | 3 |
| 25 | | Perfluoromethylcyclohexane | 4 |
| 26 | | Tetramethylurea | 16 |
| 27 | | Dimethyl carbonate | 20 |
| 28 | | Propylene carbonate | 24 |
| 29 | | Ethylene carbonate | 32 |

[Table 2]

| Experimental No. | Catalyst | Reaction medium | TOF [$h^{-1}$] |
|---|---|---|---|
| 30 | Compound 1a | Hexafluoroisopropanol | 60 |
| 31 | Compound 1b | | 65 |
| 32 | Compound 1c | | 69 |
| 33 | Compound 1d | | 64 |
| 34 | Compound 1e | | 55 |
| 35 | Compound 1f | | 43 |
| 36 | Compound 1g | | 53 |
| 37 | Compound 1h | | 67 |
| 38 | Compound 1i | | 74 |
| 39 | Compound 2a | | 70 |
| 40 | Compound 2b | | 65 |
| 41 | Compound 2c | | 62 |
| 42 | Compound 2d | | 61 |
| 43 | Compound 2e | | 34 |
| 44 | Compound 3a | | 63 |
| 45 | Compound 3b | | 42 |
| 46 | Compound 3c | | 33 |
| 47 | Compound 3d | | 53 |
| 48 | Compound 4a | | 34 |
| 49 | Compound 4b | | 30 |
| 50 | Compound 4c | | 20 |
| 51 | Compound 4d | | 16 |
| 52 | Compound 5a | | 21 |
| 53 | Compound 5b | | 43 |
| 54 | Compound 5c | | 17 |
| 55 | Compound 5d | | 15 |
| 56 | Compound 6a | | 19 |
| 57 | Compound 6b | | 18 |
| 58 | Compound 7a | | 41 |
| 59 | Compound 7b | | 39 |
| 60 | Compound 8a | | 32 |
| 61 | Compound 8b | | 43 |
| 62 | Compound 9a | | 42 |
| 63 | Compound 9b | | 38 |
| 64 | Compound 10a | | 68 |
| 65 | Compound 10b | | 74 |
| 66 | Compound 10c | | 65 |
| 67 | Compound 10d | | 76 |
| 68 | Compound 10e | | 64 |

As shown in Table 1, the TOF was highest when the reaction medium was water. However, when the inside of the autoclave returns to atmospheric pressure, dewatering of the produced formic acid starts due to the action of the catalyst, and after 2 hours, most of the formic acid is decomposed into hydrogen and carbon dioxide. That is, the formic acid obtained by the method for producing the formic acid in which the reaction medium is water is not practical because of instability. In order to suppress the decomposition of the formic acid, it is necessary to remove the catalyst or the produced formic acid from the autoclave. However, the removal process causes the producing process or the producing apparatus of formic acid to be complicated. On the other hand, as listed in Table 2, the formic acid was obtained at a favorable rate even when the ligand of iridium in the catalyst was changed. Furthermore, even when the counter anion or the anion coordinated to iridium in the catalyst was changed, the formic acid was obtained at a favorable rate.

Chemical structural formulae of the organic iridium complex used in Example 1 are shown below.

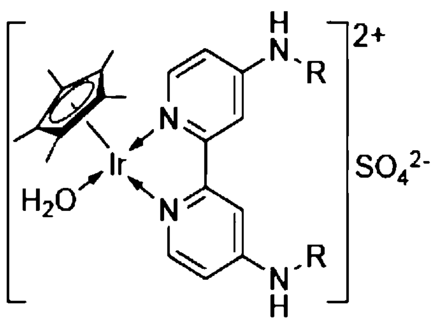

Compound 13a (R is methyl group)

Compound 13b (R is hexyl group)

Compound 13c (R is aminohexyl group)

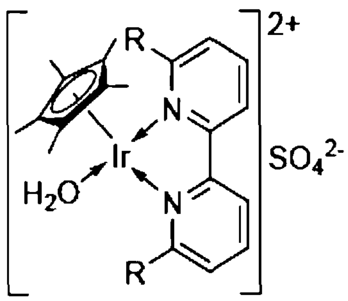

Compound 14a (R is methyl group)

Compound 14b (R is hydroxyl group)

Compound 14c (R is methoxy group)

Compound 14d (R is methylamino group)

Compound 14e (R is aminohexylamino group)

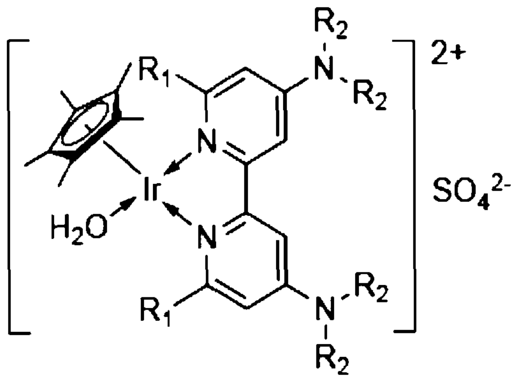

Compound 15 ($R_1$ is hydroxyl group and $R_2$ is methyl group)

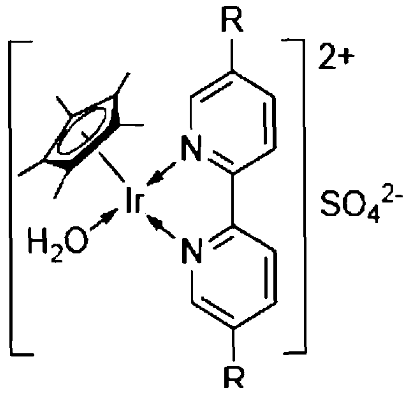

Compound 16a (R is methyl group)

Compound 16b (R is hydroxyl group)

Compound 16c (R is methoxy group)

Compound 16d (R is methylamino group)

Compound 16e (R is aminohexylamino group)

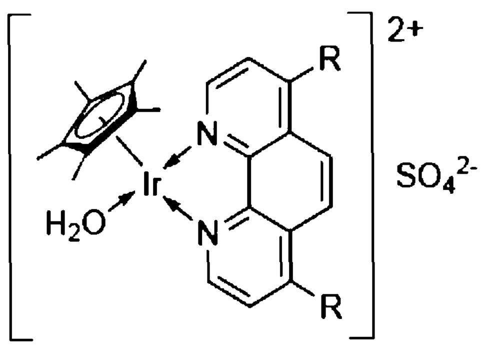

Compound 17a (R is hydroxyl group)

Compound 17b (R is methoxy group)

Compound 17c (R is aminomethyl group)

Compound 17d (R is aminohexylamino group)

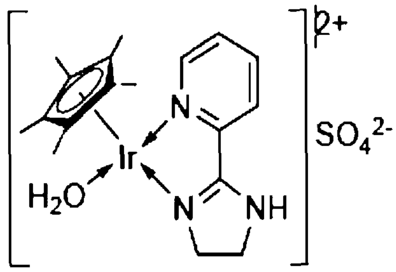

Compound 18

The compounds 13a to 13c, the compounds 14a to 14e, the compound 15, the compounds 17a to 17d, and the compound 18 were synthesized based on the description in Inorganic Chemistry, 2020, 59, 7, 4191-4199. For the compounds 16a to 16e, a ligand was synthesized based on the description in Tetrahedron Letters, 2005, 46, 2197-2199, and then a complex was synthesized based on the description in Inorganic Chemistry, 2020, 59, 7, 4191-4199.

A chemical structure formula of the organic ruthenium complex used in Example 1 is shown below.

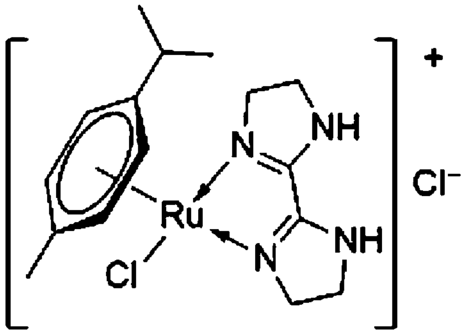

Compound 21

The compound 19 was synthesized based on the description in International Journal of Hydrogen Energy, 2019, 44 (53), 28507-28513.

[Table 3]

| Experimental No. | Catalyst | Reaction medium | TOF ($h^{-1}$) |
|---|---|---|---|
| 69 | Compound 13a | Hexafluoroisopropanol | 135 |
| 70 | Compound 13b | | 121 |
| 71 | Compound 13c | | 130 |
| 72 | Compound 14a | | 70 |
| 73 | Compound 14b | | 103 |
| 74 | Compound 14c | | 101 |
| 75 | Compound 14d | | 78 |
| 76 | Compound 14e | | 68 |
| 77 | Compound 15 | | 38 |
| 78 | Compound 16a | | 30 |
| 79 | Compound 16b | | 10 |
| 80 | Compound 16c | | 8 |
| 81 | Compound 16d | | 35 |
| 82 | Compound 16e | | 32 |
| 83 | Compound 17a | | 35 |
| 84 | Compound 17b | | 28 |
| 85 | Compound 17c | | 22 |
| 86 | Compound 17d | | 20 |
| 87 | Compound 18 | | 64 |
| 101 | Compound 21 | | 20 |

As listed in Table 3, the formic acid was obtained at a favorable rate even when the ligand of iridium in the catalyst was changed. Furthermore, even when the counter anion or the anion coordinated to iridium in the catalyst was changed, the formic acid was obtained at a favorable rate.

In addition, even when the metal in the catalyst was changed from iridium to ruthenium, the formic acid was obtained at a favorable rate.

Example 2: Immobilized catalyst

Formic acid was produced from hydrogen and carbon dioxide in the same manner as in Example 1 using a reaction medium of hexafluoroisopropanol, except that the organic iridium complex catalyst was changed to the following immobilized catalyst 10a or the following immobilized catalyst 10b. The results are listed in Table 3 below. The immobilized catalyst 10a or the immobilized catalyst 10b was obtained by supporting the organic iridium complex used in Example 1 on branched polyethyleneimine having a molecular weight of 10,000 or more.

That is, branched polyethyleneimine (Sigma-Aldrich Co., LLC, branched polyethyleneimine, molecular weight: 10,000) and a ligand of 4,4'-dichloro-2,2'-bipyridine were reacted in an autoclave to obtain a solid polymer in which the ligand was immobilized. Thereafter, an organic iridium complex $[Cp^{*}Ir(H_2O)_3](SO_4)$ ($Cp^{*}$ is a pentamethylcyclopentadienyl group (the same applies hereinafter)) was added thereto and reacted, thereby obtaining an immobilized catalyst 10a or an immobilized catalyst 10b in which the compound 1a or the compound 1b was immobilized on polyethyleneimine. In the chemical formulae representing the immobilized catalyst 10a or the immobilized catalyst 10b, m, m', n, n', n", and n‴ are natural numbers.

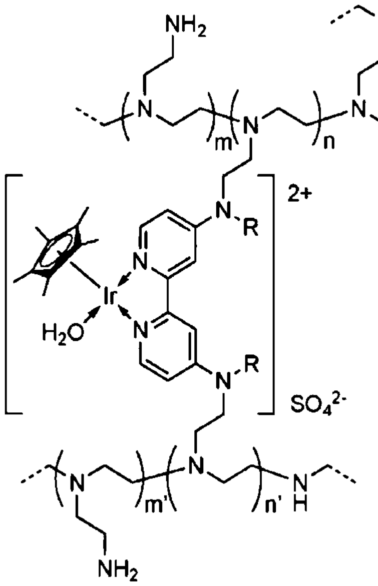

or

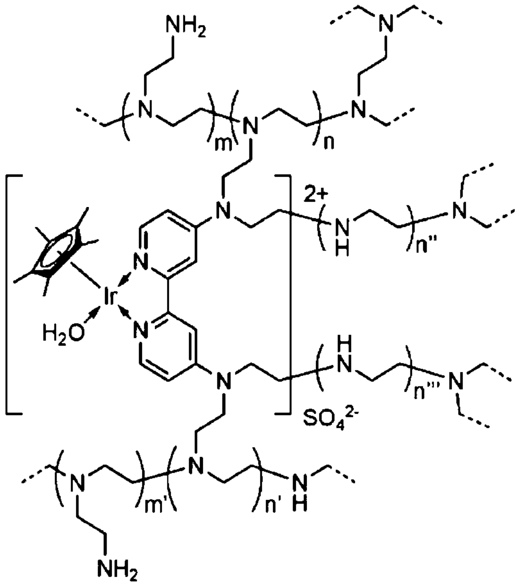

Immobilized catalyst 10a (R is hydrogen atom)
Immobilized catalyst 10b (R is methyl group)

In addition, formic acid was produced from hydrogen and carbon dioxide in the same manner as in Example 1 using a reaction medium of hexafluoroisopropanol, except that the organic iridium complex catalyst was changed to the following immobilized catalyst 11a. The results are listed in Table 3 below. The immobilized catalyst 11a was produced based on the description in R. Kanega, M. Z. Erterm, N. Onishi, D. J. Szalda, E. Fujita, Y. Himeda, Organometallics, 2020, 39, 1519-1531. In the chemical formula representing the immobilized catalyst 11a, m' and n' are natural numbers.

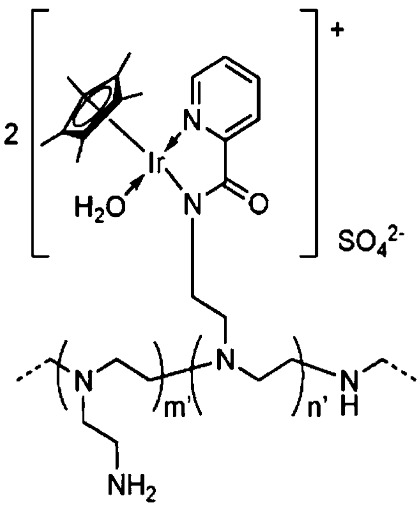

or

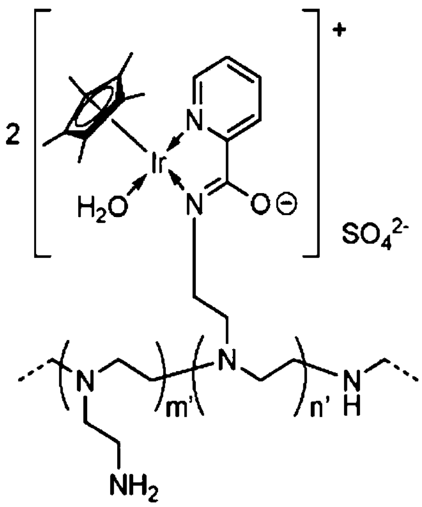

Immobilized catalyst 11a

Furthermore, formic acid was produced from hydrogen and carbon dioxide in the same manner as in Example 1 using a reaction medium of hexafluoroisopropanol, except that the organic iridium complex catalyst was changed to the following immobilized catalyst 12. The results are listed in Table 3 below. The immobilized catalyst 12 was synthesized by the following method. That is, polyacrylic acid (manufactured by FUJIFILM Wako Pure Chemical Corporation, molecular weight: 250,000) and a ligand of 4,4'-dichloro-2,2'-bipyridine were reacted in an autoclave to obtain a solid polymer in which the ligand was immobilized. Thereafter, an organic iridium complex $[Cp^*Ir(H_2O)_3](SO_4)$ was added thereto and reacted, thereby obtaining an immobilized catalyst 12 in which the compound 1a was immobilized on polyacrylic acid. In the chemical formulae representing the immobilized catalyst 12, m, m', n, n', n", and n‴ are natural numbers.

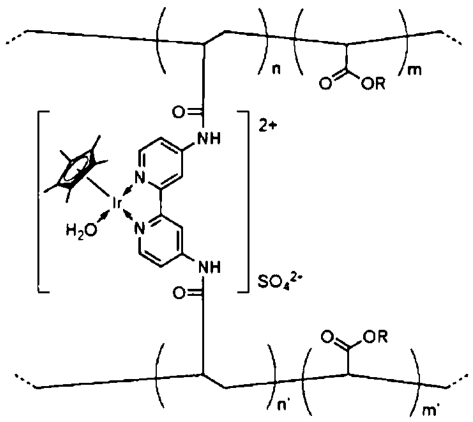

or

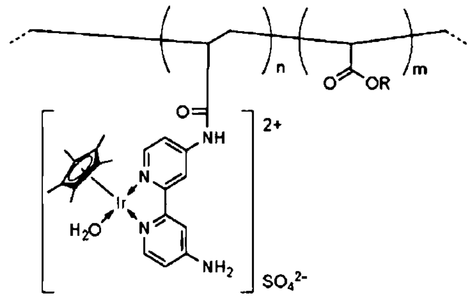

Immobilized catalyst 12

[Table 4]

| Experimental No. | Catalyst | Reaction medium | TOF [$h^{-1}$] |
|---|---|---|---|
| 88 | Immobilized catalyst 10a | Hexafluoroisopropanol | 25 |
| 89 | Immobilized catalyst 10b | | 22 |
| 90 | Immobilized catalyst 11a | | 11 |
| 91 | Immobilized catalyst 12 | | 15 |

As listed in Table 4, even when the immobilized catalyst in which an organic iridium complex was supported by a polymer compound was used, the formic acid was obtained at a favorable rate.

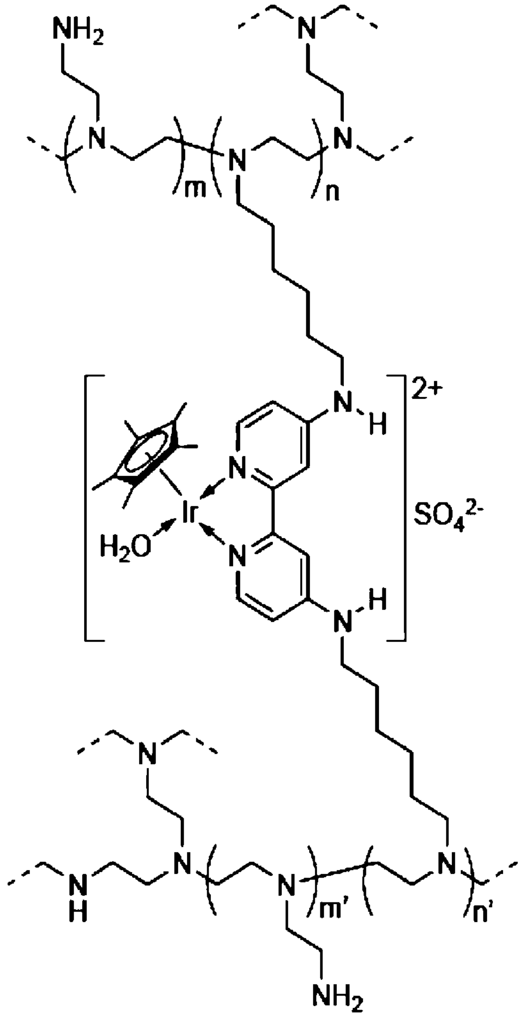

Immobilized catalyst 19

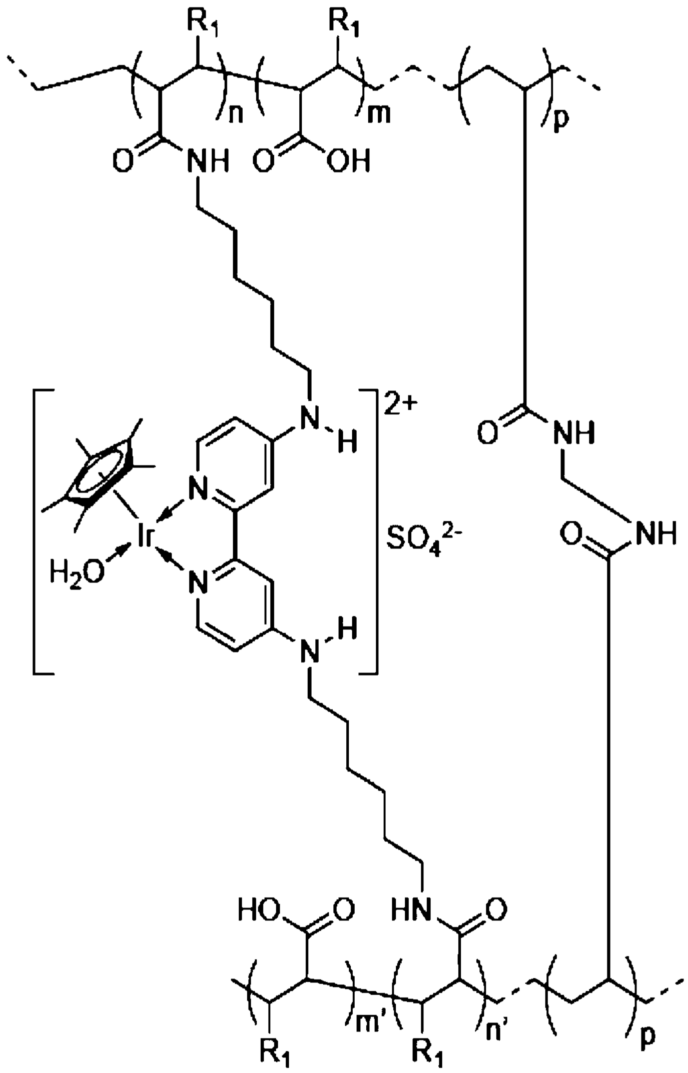

Immobilized catalyst 20a ($R_1$ is hydrogen)
Immobilized catalyst 20b ($R_1$ is methyl group)

The immobilized catalyst 19 was synthesized according to K. Sawahara, S. Takana, T. Kodaira, R. Kanega, H. Kawanami, ChemSusChem, 2024, 17, e202301282.

The immobilized catalyst 20a was synthesized by the following method.

A solution of methanol/water = 1:1 was prepared, and acrylic acid, methylenebisacrylamide as a crosslinking agent, and sodium dodecyl sulfate as a surfactant were dissolved in the solution. Thereafter, azobisisobutyronitrile was added thereto, and the mixture was heated at 80°C for 10 minutes to precipitate a polymer. The precipitated polymer was filtered, washed three times with a methanol/water = 1:1 solution, and dried. The polymer obtained by the drying was dispersed in dichloromethane, $N^1$,$N^{1'}$-([2,2'-bipyridine]-4,4'-diyl)bis(hexane-1,6-diamine) was added thereto, and subsequently 1-[3-(dimethylamino)propyl]-3-ethylcarbodiimide as a dewatering agent was added thereto and reacted at room temperature for 12 hours. The solid obtained after the reaction was dispersed in water, and an organic iridium complex [pentamethylcyclopentadienyl iridium $(H_2O)_3$]sulfate was added thereto and reacted, thereby obtaining an immobilized catalyst 20a.

An immobilized catalyst 20b was obtained in the same manner as the immobilized catalyst 20a, except that the acrylic acid was changed to methacrylic acid.

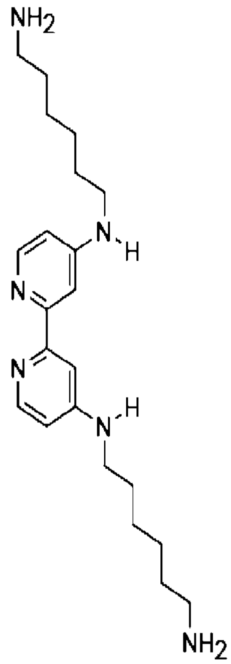

$N^1$,$N^{1'}$-([2,2'-bipyridine]-4,4'-diyl)bis(hexane-1,6-diamine)

[Table 5]

| Experimental No. | Catalyst | Reaction medium | TOF ($h^{-1}$) |
|---|---|---|---|
| 92 | Immobilized catalyst 19 | Hexafluoroisopropanol | 43 |
| 93 | Immobilized catalyst 20a | | 25 |
| 94 | Immobilized catalyst 20b | | 13 |

As listed in Table 5, even when the immobilized catalyst in which an organic iridium complex was supported by a polymer compound was used, the formic acid was obtained at a favorable rate.

Example 3: Solid catalyst

Formic acid was produced from hydrogen and carbon dioxide in the same manner as in Example 1 using a reaction medium of hexafluoroisopropanol, except that the organic iridium complex catalyst was changed to a solid catalyst listed in Table 4 below. The results are listed in Table 4. Pd/C (Pd/C manufactured by N.E. Chemcat Co., Ltd., STD type (Pd 5%) (containing water)) is a solid catalyst in which palladium is supported on carbon at a support rate of 5% by mass. A content of water was 10% by mass or less. The support rate (%) of the catalytic metal is calculated by mass of supported catalytic metal/mass of support × 100.

Pd-Au/C is a solid catalyst in which an alloy containing palladium and gold in the same mass (1:1) is supported on carbon at a support rate of 5% by mass. Pd-Ag/C is a solid catalyst in which an alloy containing palladium and silver in the same mass (1:1) is supported on carbon at a support rate of 5% by mass. Pd/rGO is a solid catalyst in which a material obtained by converting a carbon material (VULCAN XC72 Carbon Black (the same applies hereinafter)) into graphene oxide is used as a support, and palladium is supported on the support at a support rate of 5% by mass. Pd/PDA/rGO is a solid catalyst in which a material obtained by converting the carbon material into graphene oxide is used as a support, para-aminobenzene is supported on the support, and then palladium is further supported on the support at a support rate of 5% by mass. A content of water was 10% by mass or less in any cases.

Pd-Au/PDA/rGO is a solid catalyst in which a material obtained by converting the carbon material into graphene oxide is used as a support, para-aminobenzene is supported on the support, and then an alloy containing palladium and gold in the same mass (1:1) is further supported on the support at a support rate of 5% by mass. A content of water was 10% by mass or less. Pd-Au/C, Pd-Ag/C, Pd/rGO, Pd/PDA/rGO, and Pd-Au/PDA/rGO were manufactured based on the description in Heng Zhong, Masayuki Iguchi, Fu-Zhang Song, Maya Chatterjee, Takayuki Ishizaka, Ikuhiro Nagao, Qiang Xu, Hajime Kawanami, Sustainable Energy & Fuels, 1, 1049, 2017.

[Table 6]

| Experimental No. | Catalyst | Reaction medium | TOF [$h^{-1}$] |
|---|---|---|---|
| 95 | Pd/C | Hexafluoroisopropanol | 5 |
| 96 | Pd-Au/C | | 10 |
| 97 | Pd-Ag/C | | 6 |
| 98 | Pd/rGO | | 7 |
| 99 | Pd/PDA/rGO | | 10 |
| 100 | Pd-Au/PDA/rGO | | 17 |

As shown in Table 6, even when the solid catalyst in which a catalytic metal was supported on a support was used, the formic acid was obtained at a favorable rate.

Example 4: Change in reaction pressure

Formic acid was produced from hydrogen and carbon dioxide in the same manner as in Experimental No. 6 of Example 1, except that the partial pressure of the carbon dioxide in the autoclave was changed from 1 MPa to 15 MPa. The results are shown in FIG. 1. As shown in FIG. 1, when the reaction pressure (total pressure) was 1 MPa to 3 MPa, the production rate of formic acid (TOF = 320 $h^{-1}$) was the highest. In addition, the production rate of formic acid was low in the subcritical region at 6 MPa, 7 MPa, and 8 MPa. In addition, as shown in FIG. 1, even when the reaction pressure (total pressure) of carbon dioxide was 8 MPa to 13 MPa, which was equal to or higher than the critical pressure of the carbon dioxide, the production rate of formic acid was increased, and the maximum TOF = 370 $h^{-1}$ was obtained, which was the highest. Therefore, when hydrogen and carbon dioxide in a subcritical state or a supercritical state were reacted using the catalyst according to the present application, the formic acid was efficiently produced.

Example 5: Change in reaction time

Formic acid was produced from hydrogen and carbon dioxide in the same manner as in Experimental No. 6 of Example 1, except that the partial pressure of the hydrogen in the autoclave was set to 0.5 MPa, the partial pressure of the carbon dioxide was set to 12 MPa, the total pressure was set to 12.5 MPa, and the reaction time was changed from 1 hour to 100 hours. Using the compound 1b (0.2 μmol) and the amount of formic acid produced (mol), a catalytic rotation speed (TON value) was calculated according to the following expression. The results are shown in FIG. 2. As shown in FIG. 2, the reaction generally reached equilibrium in a reaction time of approximately 48 hours, the amount of formic acid produced was constant, and the TON value was also generally 2,000 or less.

TON value = Amount of formic acid produced (mol/10 mL)/0.2 μmol of amount of catalyst

Example 6: Change in reaction temperature

Formic acid was produced from hydrogen and carbon dioxide in the same manner as in Example 5, except that the reaction time was set to 2 hours and the reaction temperature was changed from 30°C to 120°C. The TOF at reaction temperatures of 30°C, 40°C, 50°C, 80°C, 100°C, and 120°C were 184 $h^{-1}$, 202 $h^{-1}$, 368 $h^{-1}$, 454 $h^{-1}$, 575 $h^{-1}$, and 575 $h^{-1}$. As the reaction temperature was higher, the production rate of formic acid was higher; but the TOF value was constant at 100°C or higher, and thus the improvement in the production rate of formic acid was not observed.

Example 7: Examination of optimum ratio of hydrogen and carbon dioxide and total pressure

Under the same conditions as in Example 5, formic acid was produced by setting the total pressure to 12.5 MPa, changing the hydrogen pressure from 0.5 MPa to 7 MPa, and simultaneously changing the carbon dioxide pressure from 12 MPa to 5.5 MPa. The results are shown in FIG. 3. As a result, a favorable TON value was exhibited in a range of generally 3 MPa of hydrogen and 9.5 MPa of carbon dioxide to 7 MPa of hydrogen and 5.5 MPa of carbon dioxide; and particularly, when the pressures of hydrogen and carbon dioxide were substantially the same, the amount of formic acid produced increased together with the highest production rate. Furthermore, under the condition that the pressure ratio of hydrogen and carbon dioxide was 1:1, the conditions were examined from 1 MPa to 15 MPa. The results are shown in FIG. 4. It was found that the production rate and the production amount of formic acid increased with the increase in pressure, and further

increased with a high pressure. When the reaction time was extended to 96 hours, the TON value increased to the maximum value of 6,182.

## Claims

**1.** A formic acid production method comprising:

producing formic acid from hydrogen and carbon dioxide in a presence of a catalyst, using a reaction medium in which a content of water is 10% by mass or less or without using a reaction medium,
wherein the catalyst contains an organic iridium complex or an organic ruthenium complex, and
a partial pressure of the hydrogen is 0.05 MPa or more and a partial pressure of the carbon dioxide is 0.05 MPa or more.

**2.** A formic acid production method comprising:

producing formic acid from hydrogen and carbon dioxide in a presence of a catalyst, using a reaction medium in which a content of water is 10% by mass or less or without using a reaction medium,
wherein the catalyst contains a support and one or more metals selected from the group consisting of chromium, manganese, iron, cobalt, nickel, copper, zinc, molybdenum, ruthenium, rhodium, palladium, silver, tungsten, rhenium, osmium, iridium, platinum, and gold, the metals being supported on the support, and
a partial pressure of the hydrogen is 0.05 MPa or more and a partial pressure of the carbon dioxide is 0.05 MPa or more.

**3.** The formic acid production method according to Claim 1,

wherein the organic iridium complex and the organic ruthenium complex are represented by General Formula (1),

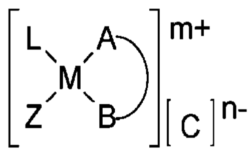

(1)

M is iridium or ruthenium, A and B are each independently a ligand containing nitrogen, carbon, oxygen, or sulfur as a coordinating atom to M, A and B may be the same or different from each other, A⌒B is a bidentate ligand coordinated to M, L is an aromatic anion ligand or an aromatic ligand, an aromatic ring of the aromatic anion ligand or the aromatic ligand may have one or more substituents, Z is an optional ligand or is not present, $[C]^{n-}$ is a counterion when $n \neq 0$, or is not present when $n = 0$, and m and n are integers.

**4.** The formic acid production method according to Claim 3,

wherein the organic iridium complex and the organic ruthenium complex are represented by General Formula (2),

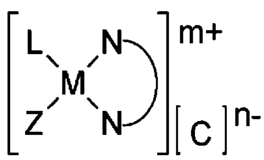

(2)

N⌒N is a bidentate ligand in which a coordinating atom to M is nitrogen.

**5.** The formic acid production method according to Claim 4,
wherein L is a pentamethylcyclopentadienyl ligand or a p-cymene ligand.

**6.** The formic acid production method according to Claim 5,

wherein the organic iridium complex and the organic ruthenium complex are represented by any one of General Formulae (9) to (12) and General Formulae (9') to (12'),

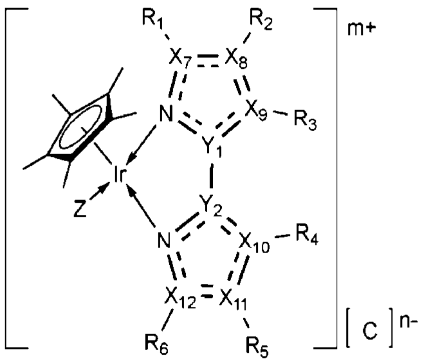

(9)

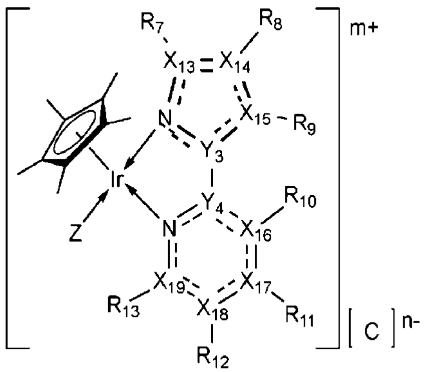

(10)

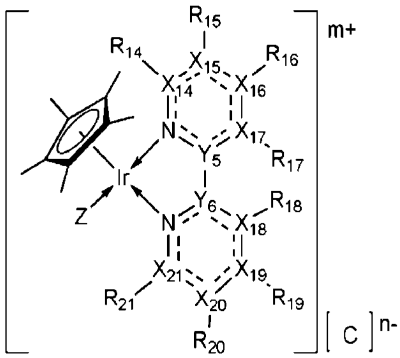

(11)

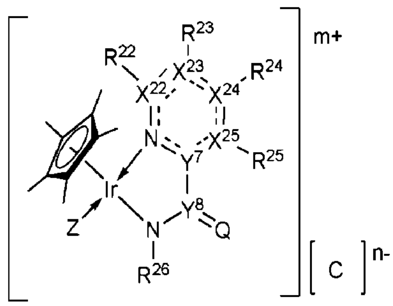

(12)

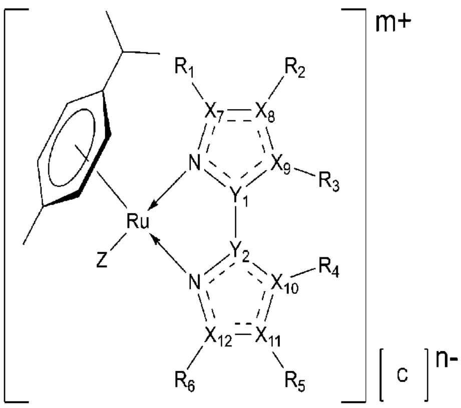

(9')

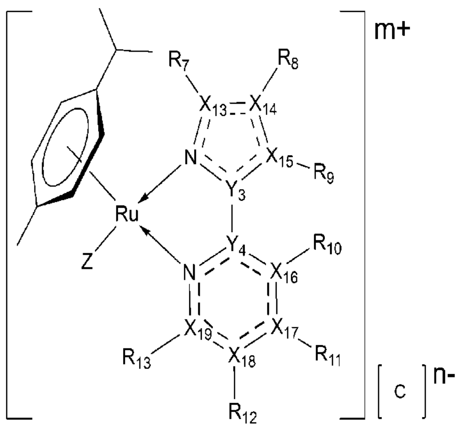

(10')

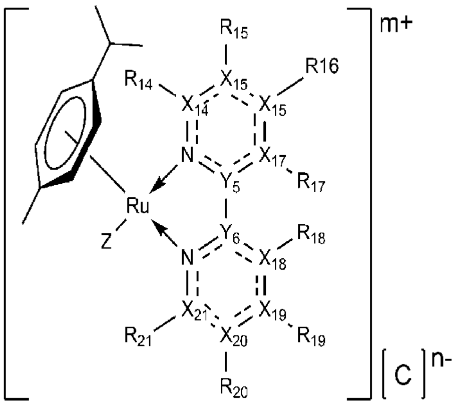

( 11' )

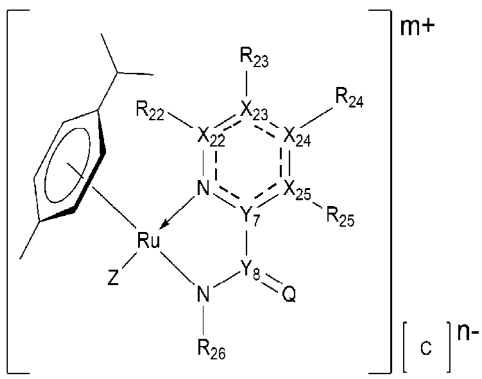

( 12' )

$X_7$ to $X_{21}$ and $X^{22}$ to $X^{25}$ are each independently nitrogen, carbon, oxygen, or sulfur, when $X_h$ (h is an integer of 7 or more and 21 or less) or $X^i$ (i is an integer of 22 or more and 25 or less) is oxygen or sulfur, $R_j$ (j is an integer of 1 or more and 21 or less) and $R^k$ (k is an integer of 22 or more and 25 or less), bonded to $X^h$ or $X^i$, are not present, $Y_1$ to $Y_6$, $Y^7$, and $Y^8$ are each independently nitrogen or carbon, $R_1$ to $R_{21}$ and $R^{22}$ to $R^{26}$ are each independently a hydrogen atom, a hydroxy group, a nitro group, a halogen group, a carboxyl group, a sulfo group, or an alkyl group, an alkoxy group, an alkylthio group, an amino group, an alkylamino group, an amide group, an ester group, or a phenyl group, which may have one or more substituents, adjacent $R_1$ to $R_{21}$ and $R^{22}$ to $R^{26}$ may form a ring, a heterocyclic ring may be an aromatic ring or a non-aromatic ring, and Q in General Formulae (12) and (12') is oxygen, sulfur, or selenium.

**7.** The formic acid production method according to Claim 6,

wherein an iridium-containing moiety constituting the organic iridium complex is represented by any one of General Formulae (13) to (19), and
a ruthenium-containing moiety constituting the organic ruthenium complex is represented by any one of General Formulae (13') to (19'),

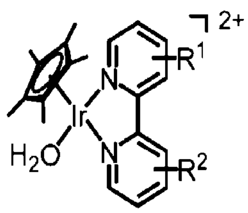

(13)

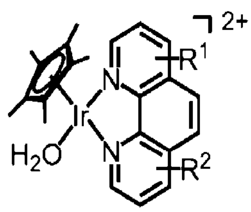

(14)

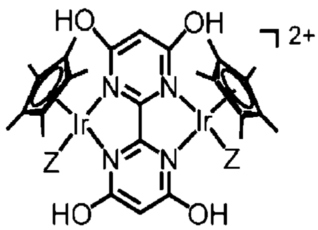

(15)

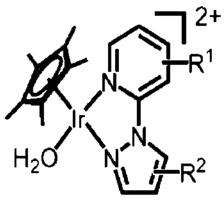

(16)

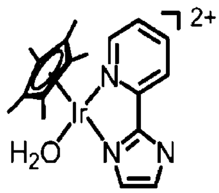

(17)

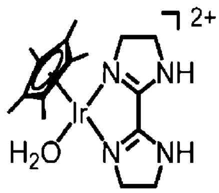

(18)

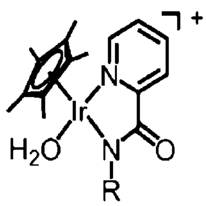

(19)

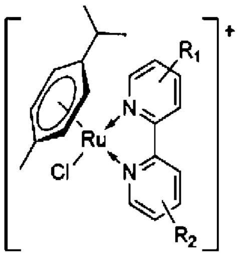

( 13' )

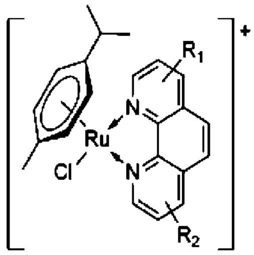

( 14' )

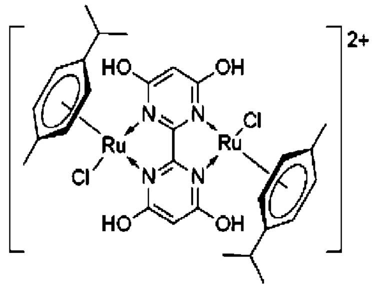

( 15' )

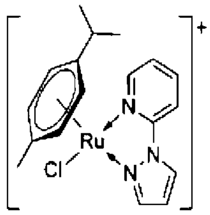

( 16' )

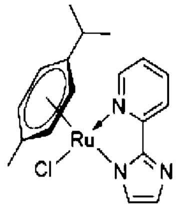

( 17' )

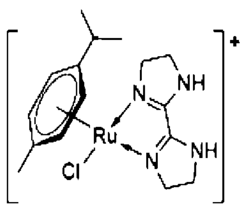

( 18' )

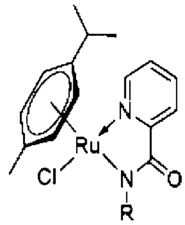

( 19' )

R, $R^1$, and $R^2$ are each independently a hydrogen atom, a hydroxy group, a nitro group, a halogen group, a carboxyl group, a sulfo group, or an alkyl group, an alkoxy group, an alkylthio group, an amino group, an alkylamino group, an amide group, an ester group, or a phenyl group, which may have one or more substituents, and adjacent R, $R^1$, and $R^2$ may form a ring.

**8.** The formic acid production method according to any one of Claim 1 and Claims 3 to 7,
wherein the organic iridium complex or the organic ruthenium complex is supported by a polymer compound.

**9.** The formic acid production method according to any one of Claims 1 to 7,
wherein the partial pressure of the carbon dioxide is 1 MPa or more and 6 MPa or less, or 8MPa or more and 13 MPa or less.

**10.** The formic acid production method according to Claim 9,
wherein the formic acid is produced from the hydrogen and the carbon dioxide at a reaction temperature of 30°C or higher and 100°C or lower.

FIG. 1

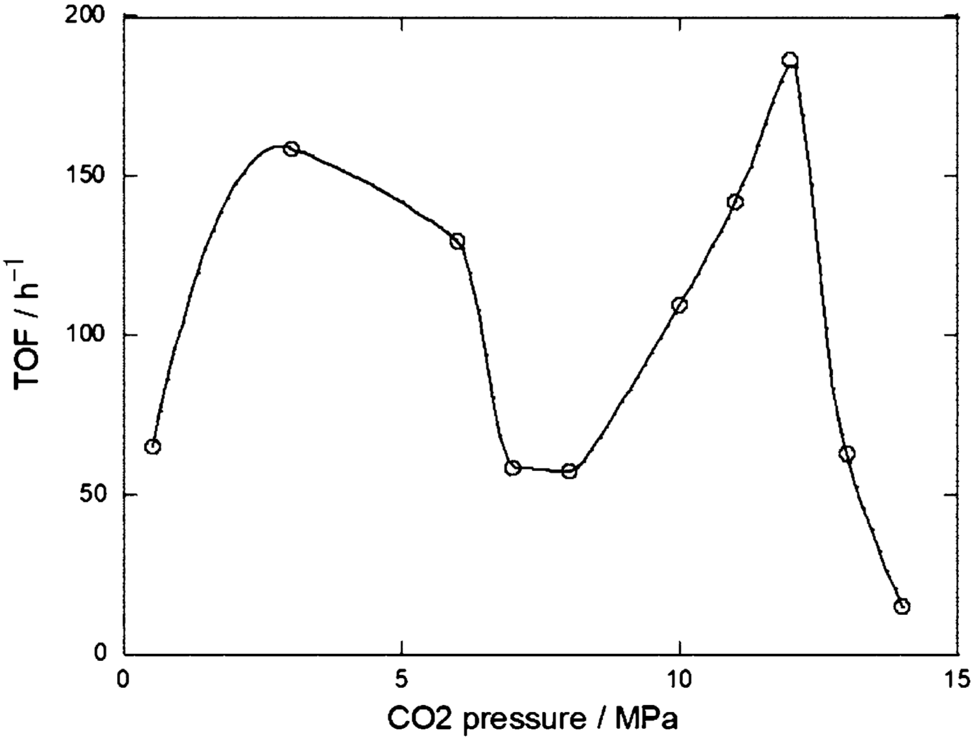
200
150
100
50
0
TOF / $h^{-1}$
0
5
10
15
CO2 pressure / MPa

FIG. 2

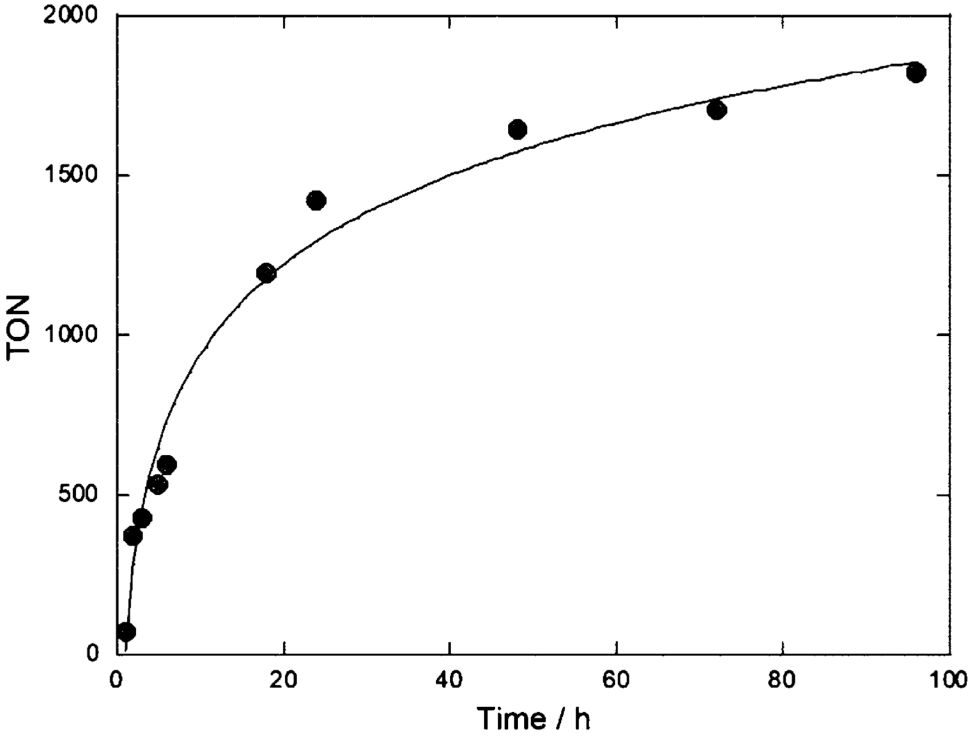
2000
1500
1000
500
0
TON
0
20
40
60
80
100
Time / h

FIG. 3

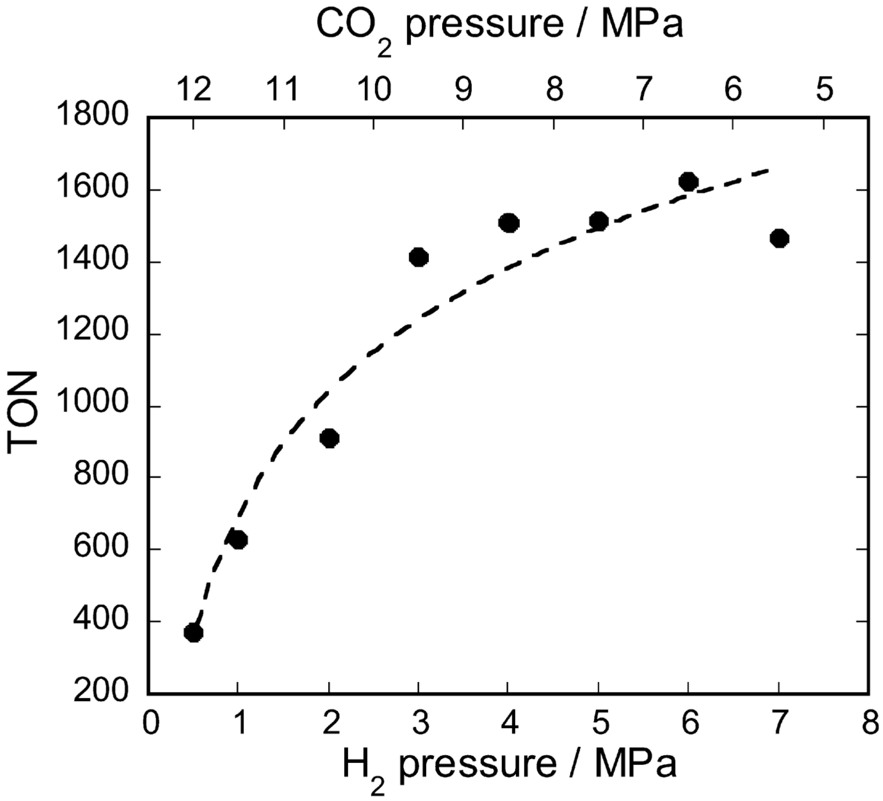
CO2 pressure / MPa
12
11
10
9
8
7
6
5
1800
1600
1400
1200
1000
800
600
400
200
TON
0
1
2
3
4
5
6
7
8
H2 pressure / MPa

FIG. 4

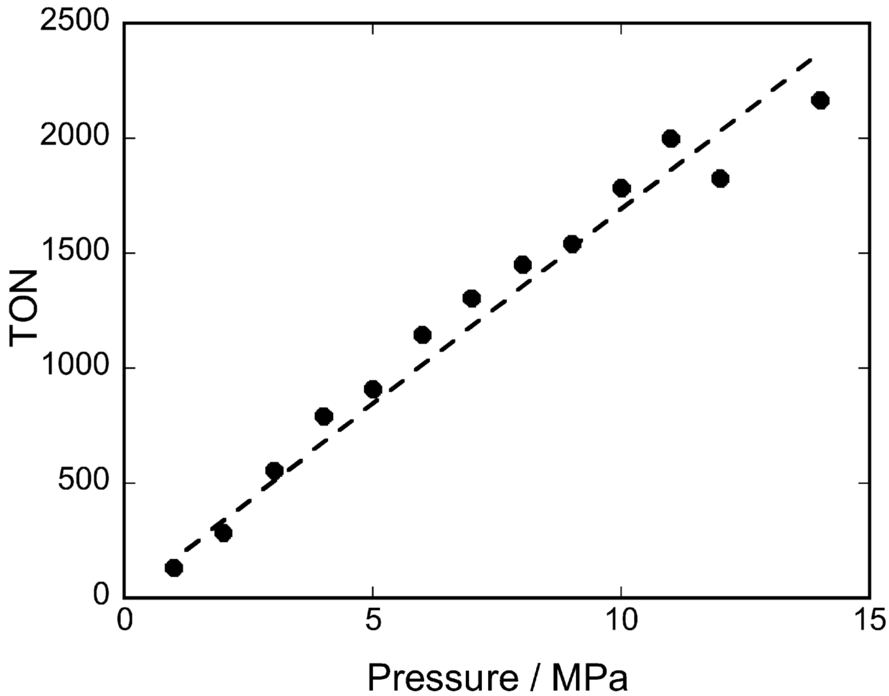
2500
2000
1500
1000
500
0
TON
0
5
10
15
Pressure / MPa

## INTERNATIONAL SEARCH REPORT

International application No.
**PCT/JP2024/002489**

### A. CLASSIFICATION OF SUBJECT MATTER

***C07C 51/00***(2006.01)i; ***B01J 31/22***(2006.01)i; ***C07B 61/00***(2006.01)i; ***C07C 53/02***(2006.01)i
FI: C07C51/00; B01J31/22 Z; C07C53/02; C07B61/00 300

According to International Patent Classification (IPC) or to both national classification and IPC

### B. FIELDS SEARCHED

Minimum documentation searched (classification system followed by classification symbols)

C07C51/00; B01J31/22; C07B61/00; C07C53/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X<br><br>Y | WO 2020/184254 A1 (NITTO DENKO CORPORATION) 17 September 2020 (2020-09-17) paragraphs [0001], [0011], [0014], [0015], [0018], [0023], [0024], [0035]-[0054] | 1, 2, 9, 10<br><br>1-10 |
| Y | JP 2013-193983 A (NATIONAL INSTITUTE OF ADVANCED INDUSTRIAL SCIENCE AND TECHNOLOGY) 30 September 2013 (2013-09-30) paragraphs [0012], [0013], [0033], [0034], [0036], [0043], [0045], [0047]-[0049], complex ligand represented by formula (1) | 1-10 |
| Y | JP 2018-199103 A (THE NATIONAL INSTITUTE OF ADVANCED INDUSTRIAL SCIENCE AND TECHNOLOGY) 20 December 2018 (2018-12-20) paragraphs [0010], [0034], [0035], [0037], [0039], [0043], [0046], [0047], complex 1, 7, 10, 22 | 1-10 |
| Y | JP 2015-502914 A (BROOKHAVEN SCIENCE ASS LLC ) 29 January 2015 (2015-01-29) paragraphs [0009], [0010], [0017]-[0019], [0023], [0030]-[0032], catalyst of formula II | 1-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 April 2024** | **16 April 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
**PCT/JP2024/002489**

C. DOCUMENTS CONSIDERED TO BE RELEVANT

| Category* | Citation of document, with indication, where appropriate, of the relevant passages | Relevant to claim No. |
|---|---|---|
| A | KANEGA, R. et al. $CO_2$ Hydrogenation Catalysts with Deprotonated Picolinamide Ligands. ACS Catalysis. 21 August 2017, vol. 7, no. 10, pp. 6426-6429, doi:10.1021/acscatal.7b02280 schemes 1-3 | 1-10 |

Form PCT/ISA/210 (second sheet) (July 2022)

**INTERNATIONAL SEARCH REPORT**
**Information on patent family members**

International application No.
**PCT/JP2024/002489**

| Patent document cited in search report | Publication date (day/month/year) | Patent family member(s) | Publication date (day/month/year) |
|---|---|---|---|
| WO 2020/184254 A1 | 17 September 2020 | US 2022/0177402 A1<br>paragraphs [0001], [0016], [0022]-[0025], [0028], [0046]-[0051], [0072]-[0113]<br>EP 3936498 A1 | |
| JP 2013-193983 A | 30 September 2013 | (Family: none) | |
| JP 2018-199103 A | 20 December 2018 | (Family: none) | |
| JP 2015-502914 A | 29 January 2015 | US 2014/0299817 A1<br>paragraphs [0007]-[0011], [0024]-[0043], [0064]-[0069], [0085]<br>EP 2755966 A2<br>WO 2013/040013 A2 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2023010364 A
- JP 6502091 B
- JP 7370040 B


### Non-patent literature cited in the description


- **H. KAWANAMI** ; **M. IGUCHI** ; **Y. HIMEDA**. *Inorganic Chemistry*, 2020, vol. 59 (7), 4191-4199
- Development of Energy Saving Hydrogen Boosting System Using Renewable Energy. *FY 2015 Results Report New Energy Venture Technology Innovation Project New Energy Venture Technology Innovation Project (Fuel Cell and Storage Battery)*, 14 November 2017
- **D. WEI** ; **R. SANG** ; **P. SPONHOLZ** ; **H. JUNGE** ; **M. BELLER**. *Nature Energy*, 2022, vol. 7, 438-448
- **W. L. HARDY**. Formic Acid Manufacture. *Industrial and Engineering Chemistry*, 1957, vol. 49 (7), 45A-46A
- *Inorganic Chemistry*, 2020, vol. 59 (7), 4191-4199
- *Tetrahedron Letters*, 2005, vol. 46, 2197-2199
- *International Journal of Hydrogen Energy*, 2019, vol. 44 (53), 28507-28513
- **R. KANEGA** ; **M. Z. ERTERM** ; **N. ONISHI** ; **D. J. SZALDA** ; **E. FUJITA** ; **Y. HIMEDA**. *Organometallics*, 2020, vol. 39, 1519-1531
- **K. SAWAHARA** ; **S. TAKANA** ; **T. KODAIRA** ; **R. KANEGA** ; **H. KAWANAMI**. *ChemSusChem*, 2024, vol. 17, e202301282
- **HENG ZHONG** ; **MASAYUKI IGUCHI** ; **FU-ZHANG SONG** ; **MAYA CHATTERJEE** ; **TAKAYUKI ISHIZAKA** ; **IKUHIRO NAGAO** ; **QIANG XU** ; **HAJIME KAWANAMI**. *Sustainable Energy & Fuels*, 2017, vol. 1, 1049